# EUROPEAN PATENT SPECIFICATION

(11) **EP 1 838 851 B1**
(45) Date of publication and mention of the grant of the patent: **16.09.2009**
(21) Application number: 06703246.6
(22) Date of filing: 19.01.2006
(51) Int. Cl.: C12N 15/10, C12Q 1/68

(54) **POLYPEPTIDE MUTAGENESIS METHOD**
POLYPEPTIDMUTAGENESEVERFAHREN
PROCEDE DE MUTAGENESE DE POLYPEPTIDE

(30) Priority: 20.01.2005 GB 0501189
(43) Date of publication of application: 03.10.2007
(73) Proprietor: University College Cardiff Consultants Limited, Cardiff, South Glamorgan CF24 0DE (GB)
(72) Inventor: JONES, Dafydd School of Biosciences, Cardiff, South Glamorgan, CF10 3US (GB)
(74) Representative: Tombling, Adrian George
(86) International application number: PCT/GB2006/000187
(87) International publication number: WO 2006/077411

(56) References cited:
- WO-A-02/068634
- JONES D DAFYDD: "Triplet nucleotide removal at random positions in a target gene: the tolerance of TEM-1 beta-lactamase to an amino acid deletion" NUCLEIC ACIDS RESEARCH, vol. 33, no. 9, 16 May 2005 (2005-05-16), XP002371879 ISSN: 0305-1048

## Description

### FIELD OF INVENTION

The invention relates to a method for altering the amino acid sequence of a target polypeptide, by insertion, deletion or substitution of at least one amino acid in the target polypeptide.

### BACKGROUND

### Protein mutagenesis

Nature has evolved an impressive myriad of proteins to perform the functions for the fitness of an organism. Changes to gene sequences are translated into changes in the amino acid composition of the protein. Nucleotide substitution, deletion or insertion are utilised by nature during the evolutionary process (Chothia, C. et al. (2003) Science 300 1701-1703). Substitution of a single nucleotide can result in the change in character of the amino acid by altering the information that encodes the amino acid at that particular position. Selection pressure means that deletion or insertion of three nucleotides or multiples thereof are favoured as they maintain the reading frame of the gene (Taylor, M. et al. (2004) Genome Res. 14 555-566). During the process of divergent evolution, many substitution and insertion-deletion (indel) mutations result in the change in composition of the protein (Taylor, M. *et al.* (2004); Lesk, A.M. (2001) Introduction to protein architecture. Oxford University Press, Oxford; Pascarella, S. & Argos, P. (1992) J. Mol. Biol. 224 461-471). Many of these changes have profound effects on the properties of the protein especially folding, ligand or substrate binding, protein-protein interactions and temperature-dependent activity and stability. For example, the sequence variation of the immunoglobulin variable domains due to substitution mutagenesis is enhanced by amino acid deletions and insertions (de Wildt, R.M. et al. (1999) J. Mol. Biol. 294 701-710) and various substitution and indel events are observed between the structurally homologous subtilisin serine proteases, including in regions known to be important for catalysis, substrate recognition and calcium binding (Siezen, R.J. & Leunissen, J.A. (1997) Protein Sci. 6 501-523).

The introduction of random mutations throughout a target gene is a powerful method for altering the properties of a protein (see Tao, H. & Cornish, V.W. (2002) Curr. Opin. Chem. Biol. 6 858-864 and Arnold, F.H. (2001) Nature 409 253-257 for reviews). Most of the current technologies have focused on the introduction of point mutations leading to an amino acid substitution (Dalby, P.A. (2003) Curr. Opin. Struct. Biol. 13 500-505; Lutz, S. & Patrick, W.M. (2004) Curr. Opin. Biotechnol. 15 291-297 and references therein). These methods are usually restricted to the changing of one nucleotide base pair per codon, restricting the amino acid type available at that position, or rely on naturally occurring genetic diversity. Some methods have been used to introduce amino acid insertions, for example pentapeptide scanning mutagenesis (Hallet, B. et al. (1997) Nucleic Acids Res. 25 1866-1867; discussed further below) and Random Insertion and Deletion (RID) (Murakami, H. et al. (2002) Nat. Biotechnol. 20 76-81). The RID method has the potential to introduce single amino acid deletions but has currently been applied only to introduce amino acid substitutions or insertions. Furthermore, the procedure is complicated and prone to the introduction of unwanted secondary mutations (Murakami, H. *et al.* (2002)).

The insertion or deletion of a single codon is one of the most common forms of indel mutation observed in nature and illustrates its importance to the process of evolution (Taylor, M. *et al.* (2004)). Mimicking such an event *in vitro* would help our understanding of the influence of indel mutations on protein structure and function and enhance our ability to improve the properties of proteins for a particular application. Currently, the most common method of introducing indel mutations is by rational design and will thus be reliant on structural information to determine the residues to be deleted and require separate oligonucleotides for each mutation.

### Transposons

Transposons are mobile pieces of genetic information (Reznikoff et al. (1999) Biochem. Biophys. Res. Commun. 266 729-734) capable of inserting randomly into a DNA sequence. Most transposons follow a common general mechanism for this (Mizuuchi (1992) Annu. Rev. Biochem. 61 1011-1051; Craig (1995) Science 270 253-254). The transposon has a recognition sequence at each of its termini, which consists of an inverted repeat; that is the termini have identical sequences reading in opposite direction. A transposase enzyme recognises and binds to these recognition sequences to form a protein-DNA complex, which then facilitates insertion of the transposon into the target DNA by catalysing DNA cleavage and joining reactions. For example, in the case of the Mu transposon, MuA acts as the transposase. A 5 bp staggered cut is made in the target DNA before insertion of the Mu transposon. The consequential 5 bp gap on the opposite DNA strand is filled by the host organism if required or, *in vitro,* by using the appropriate enzymes. The result is the insertion of the transposon plus the repetition of 5 bp of the target DNA either side of the transposon. In the case of the Tn5 transposon, 9 bp staggered cut is made, resulting in the repetition of 9 bp of target DNA either side of the transposon (Reznikoff *et al.* (1999); Steiniger-White et al. (2004) Curr. Opin. Struct. Biol. 14 50-57). The mini-Mu and Tn5 transposition reactions have, amongst others, been adapted for use *in vitro,* with the reaction having a very low target site preference allowing transposon insertion to occur essentially at any point in a given gene (Goryshin & Reznikoff (1998) J. Biol. Chem. 273 7367-7374; Haapa et al. (1999) Nuc. Ac. Res. 27 2777-2784).

### Restriction enzymes

Restriction endonucleases are a class of enzymes that cleave DNA upon recognising a specific nucleotide sequence. The type II enzymes are a specific class of restriction endonucleases. Their recognition sites are palindromic, partially palindromic or interrupted palindromes. Unlike the type I and III restriction endonucleases, which cleave DNA randomly, the type II enzymes cleave the DNA at specific sites, normally within the recognition sequence. The type IIS enzymes are a subtype having some features atypical of common type II enzymes. They generally recognise non-palindromic or asymmetric nucleotide sequences with at least one strand cleaved outside the recognition sequence (i.e. they are so-called "outside cutters"). One such example of a type IIS restriction endonuclease is *Mly*I, that recognises a specific, non-palindromic DNA sequence and cuts 5 bp away from the recognition sequence to generate a blunt end (5' GAGTCNNNNN↓ 3'; the recognition sequence is underlined, N signifies either G, A, T or C is allowed and the arrow shows the cleavage position). Other type II enzymes are the type IIB, IIE, IIG and IIP subtypes which share some characteristics with the type IIS, subclass. For example, some members of these subtypes are classed as outside cutters.

### Known protein mutagenesis techniques

US Patent No. 5,843,772 relates to an artificial transposon known as AT-2. Restriction enzyme recognition sites were added in order to allow the liberation of the blunt-ended transposon from a DNA vector. The restriction enzyme recognition sites are recognised by restriction enzymes which cut within the recognition sequence themselves. The patent primarily relates to methods of creating artificial transposons and inserting these into DNA sequences.

Vilen et al. (J. Virol. (2003) 77 123-134) relates to the use of transposons to map genes in a virus genome. The transposons disclosed in Vilen *et al.* are not suitable for use in a method to alter the amino acid sequence of a target polypeptide.

US Patent No. 4,830,965 relates to the introduction of restriction enzyme recognition sites to allow DNA sequences to be inserted at points within a transposon. The restriction enzyme recognition sites are not located at the termini of the transposon.

US Patent No. 5,728,551 relates to the pentapeptide scanning mutagenesis technique mentioned above and discussed in more detail below. There is mention of a proposed method of codon insertion mutagenesis, although no data is provided to indicate that the method was carried out. It is proposed to position a *Srf*I restriction enzyme recognition sequence near the termini of a transposon, the *Srf*I restriction enzyme cutting within its recognition sequence. The insertion of such a transposon into a target DNA and subsequent excision using *Srf*I would result in the target DNA having a gap as the result of the transposon excision, the termini of the gap comprising some transposon-derived nucleotides, as the result of the position of the *Srf*I cleavage of the DNA. Therefore, the specific codons which could be inserted using a given transposon would be limited, since the sequence of the inserted codon would be partly determined by the sequence of the terminus of the transposon.

Hayes et al. (Applied & Environmental Microbiology (1990) 56 202-209 relates to the use of transposons to generate gene knockouts and to use restriction enzyme sites within the transposon to map the position of gene critical to plasmid replication within a cell.

TEM-1 is a clinically important protein as it is one of the main causes of bacterial resistance to β-lactam antibiotics. Many natural variants of TEM-1 exist that have evolved to confer resistance to new, extended spectrum (ES) β-lactam antibiotics (http://www.lahey.org/Studies/temtable.asp; example references are: Matthew, M. & R.W. Hedges (1976) J. Bacteriol. 125 713-718; Chanal, C.M. et al. (1989) Antimicrob. Agents Chemother. 33 1915-1920; Goussard, S. & Courvalin, P. (1999) Antimicrob. Agents Chemother. 43 367-370). Although no naturally occurring deletion variants of TEM-1 exist, amino acid deletions have been observed in homologous β-lactamases such as SHV-9 and SHV-10 (Prinarakis, E.E. et al. (1997) Antimicrob. Agents Chemother. 41 838-840) and *S. aureus* PC1 (Zawadzke, L.E. et al. (1995) Protein Eng. 8 1275-1285) that contribute to bacterial resistance to ES β-lactams. TEM-1 has also been the focus of many protein engineering studies (Matagne, A. et al. (1998) Biochem. J. 330 (Pt 2) 581-598), including the random substitution of every amino acid to determine which amino acid residues cannot tolerate mutation (Huang, W. et al. (1996) J. Mol. Biol. 258 688-703), directed evolution (for example Camps, M. et al. (2003) Proc. Natl. Acad. Sci. U.S.A 100 9727-9732; Stemmer, W.P. (1994) Nature 370 389-391) and pentapeptide scanning mutagenesis (Hayes, F. et al. (1997) J. Biol. Chem. 272 28833-28836). The pentapeptide scanning mutagenesis method concerns the insertion of a transposon and its removal with a standard, rare cutting type II restriction enzyme such as *Not*I (5' GC↓GGCCGC 3') or *Pme*I (5' GTTT↓AAC 3'). The main shortfall of this method is that it is limited in the sequence change which can be introduced. Upon restriction digestion to remove the transposon, the 5 bp duplicated region of the target DNA, together with the segment of the transposon containing the restriction site, are always incorporated into the final, modified target DNA. Therefore, this results in the insertion of a defined set of amino acids, usually greater than 5 amino acids in length. Amino acid substitutions or deletions are not sampled, neither are less drastic insertions, such as a single amino acid.

An improvement on the above method is disclosed in US-A-2005/0074892. In the method described in that document, a transposon is inserted into a target DNA sequence, the transposon being excised using a non-Type IIS restriction enzyme, leaving the target DNA with a gap created by excision of the transposon. Each terminus of the gap comprises some transposon-derived nucleotides and the duplicated nucleotides arising from transposon insertion. The transposon used in this method is commercially available and has not been modified for use in the procedure. Furthermore, the method requires several steps, involving the sequential insertion and deletion of further, non-transposable DNA sequences, with multiple restriction endonuclease digestion steps to eventually remove the transposon-derived nucleotides from the target DNA sequence. This lengthy process eventually allows insertion, deletion or substitution of a single codon within the target DNA sequence.

WO02/068631 relates to a method of producing gene libraries wherein a defined number of juxtapositioned nucleotides are exchanged in genes and gene libraries are produced, coding for protein variants.

### Creation of "molecular switches"

The ability to design and produce molecules that can change their properties in response to a desired input will allow significant new possibilities for creating novel sensing and transducing devices. The concept of the molecular switch is well established in nature, with proteins playing the lead roles in sensing chemical signals and converting them into the appropriate cellular response (Monod et al. (1963) J. Mol. Biol. 6 306-329; Changeux & Edelstein (2005) Science 308 1424-1428). Creating proteins whose output is coupled to a desired input has the potential for a wide variety of *in vivo* and *in vitro* applications, including the creation of tailored biosensors and novel intelligent materials. While it might appear simplest to use natural protein switches, these have evolved to fulfil specific functions within a defined biological context and may not have the requisite properties for a particular application. Therefore, as a general approach for creating molecular switches, functions of normally disparate proteins can be coupled.

Natural allosteric proteins have spatially distinct regulation and active sites (Monod *et al.* (1963); Changeux & Edelstein (2005)). Binding of an effector molecule at the regulation site causes conformational changes that can rapidly and reversibly modulate protein activity directly. Ideally, any artificial allosteric protein will mimic this mechanism. Rather than re-engineer natural switches, a simpler and more effective strategy is to couple the functions of normally disparate proteins through linked conformational changes (Buskirk & Liu (2005) Chem. Biol. 338 633-641; Hahn & Muir (2005) Trends Biochem. Sci. 30 26-34; Ostermeier (2005) Prot. Eng. Des. Sel. 18 359-364). Proteins are recruited that have the desired regulatory (e.g. small molecule-dependent conformational changes) and reporter (e.g. enzymatic activity) function. The two proteins need to be linked in such a manner that the conformational events occurring in the regulation domain on binding the small molecule can be transmitted to the reporter domain to modulate the output signal. One approach to link such conformational events is to use a strategy called domain insertion, in which one protein domain is inserted within another (Doi & Yanagawa (1999) FEBS Lett. 457 1-4; Ostermeier (2005) Protein Eng. Des. Sel. 18 359-364). Thus, two shared links are created, decreasing the degrees of freedom between the two domains and intimately linking their structure to promote the transmission of any conformational changes. Domains linked in the more traditional end-to-end fashion will generally act autonomously of each other, with no communication between the two.

The key to success of this strategy is the identification of sites within a protein that permit insertions of whole domains, while retaining the function of both proteins and allowing the transmission of conformational events. Analysis of natural multi-domain proteins suggests that domain insertion is a relatively common evolutionary event (Jones et al. (1998) Protein Sci. 7 233-242; Aroul-Selvam et al. (2004) J. Mol. Biol. 338 633-641). Several protein engineering studies have also shown that proteins can tolerate large insertions, including the whole domain of another protein. However, sites that permit an insertion and allow coupling may not be obvious. For example, insertions close to the active site of the reporter protein should enhance coupling by transmitting conformational changes directly to the catalytic centre yet may be considered too deleterious to enzyme activity.

Predicting sites within a target protein that permit the insertion of a whole protein domain so as to link the functions of the two proteins is currently very difficult. To overcome this obstacle, an evolutionary approach can be taken in which one protein is randomly inserted into another. To do this at the genetic level, a single break has to be introduced at random positions in the gene that encodes for the protein to be inserted into. One such method used to generate such breaks into DNA involves the use of the non-specific endonuclease, DNasel (Guntas & Ostermeier (2004) J. Mol. Biol. 336 263-273). The problem with using DNaseI is that it is notoriously difficult to generate single cuts in DNA and digestion with this non-specific endonuclease regularly produces tandem duplications and nested deletions of varying sizes. This will lead to frameshifts, large insertions and large deletions in the protein, so reducing the quality of the library and increasing the number of variants that need to be sampled. The method of the current invention will not introduce such large deletions or insertions at the protein level, allowing the researcher to dictate the size of any linking sequence with the inserted domain. There are only three possible reading frames for the inserted gene (depending on the transposon insertion point with respect to one codon), increasing the likelihood of a correct reading frame from 1 in 6, when using DNaseI, to 1 in 3 when using the method of the invention.

### SUMMARY OF INVENTION

The current invention relates to a new method that introduces triplet nucleotide deletions or nucleotide insertions at random positions throughout a target gene. Furthermore, the technology can be altered to allow amino acid substitutions that cover the whole range of amino acid sequences at a particular position. Moreover, the technology can be adapted further to allow for the insertion of longer stretches of DNA that can encode epitopes, protein fragments or even whole protein domains.

The technology has been tested by determining the effects of amino acid indels on the TEM-1 p-lactamase, encoded for by the *bla* gene.

The new technology outlined in this application will therefore complement existing knowledge by further exploring the sequence space open to TEM-1 and the effect of such mutations on TEM-1 structure and function. Furthermore, it will validate the technologies outlined in the application by providing a suitable example of the use of the technologies.

According to a first aspect of the invention, there is provided a method for altering the amino acid sequence of a target polypeptide by altering a target DNA sequence which encodes that polypeptide, the method comprising the step of introducing a transposon into the target DNA sequence, in which the transposon comprises a first restriction enzyme recognition sequence towards each of its termini, the recognition sequence not being present in the remainder of the transposon, or in the target DNA sequence, or in a construct (for example, a plasmid or vector) comprising the target DNA sequence, the first restriction enzyme recognition sequence being recognised by a first restriction enzyme which is an outside cutter and being positioned such that the first restriction enzyme has a DNA cleavage site positioned beyond the end of the terminus of the transposon.

The term "outside cutter", as used throughout this specification, is a term known in the art which indicates a restriction enzyme which cleaves DNA outside the restriction enzyme recognition sequence. Although the majority of restriction enzymes which are outside cutters belong to the type IIS subtype, members of the IIB, IIE, IIG and IIP subtypes can also be classed as outside cutters.

The term "beyond the end of the terminus of the transposon", as used throughout this specification, indicates that the first restriction enzyme cleavage site is external to the transposon sequence, such that, when the transposon is incorporated into a target DNA sequence, the cleavage site is at a position within the target DNA sequence and not at a position within the transposon DNA sequence.

Advantageously, the invention provides a simple tool for the investigation of the impact of insertions, deletions and substitutions of one or more amino acids at points throughout a polypeptide of interest. The requirement for the first restriction enzyme recognition sequence to be recognised by an enzyme which is an outside cutter advantageously allows the insertion, deletion or substitution of a single amino acid in a target polypeptide by use of the method according to the invention. In a further advantage, the use of an enzyme which is an outside cutter, along with the positioning of the recognition sequence such that the cleavage site is beyond the end of the terminus of the transposon, allows excision of the whole transposon DNA sequence from the target DNA sequence after insertion, including nucleotides located at the termini of the transposon, without the need for additional steps to allow removal of such nucleotides. Therefore, the method of the invention is simpler, quicker and hence more economical than known methods.

For example, the method may exploit the properties of the mini-Mu transposon, a DNA element that can be accurately and efficiently inserted into a target DNA sequence *in vitro* using the MuA transposase (Haapa, S. et al. (1999) Nucleic Acids Res. 27. 2777-2784). The reaction has a very low target site preference allowing transposon insertion to occur essentially at any point in a given gene. Other transposons may also be used as the basis for this technology, for example the AT-2 artificial transposon (Devine, S.E. & Boeke, J.D. (1994) Nucleic Acids Res. 22 3765-3772) or the Tn5 transposon (Goryshin & Reznikoff (1998) J. Biol. Chem. 273 7367-7374). Surprisingly, the inventor has found that it is possible to engineer a transposon to be suitable for use in a method according to the invention, by altering the termini of the transposon without disrupting the ability of the transposase enzyme to recognise the transposon. For example, it was previously shown that mutations which change the termini of mini-Mu can have an adverse effect on the ability of MuA transposase to recognise the transposon (Goldhaber-Gordon et al. (2002) J. Biol. Chem. 277 7703-7712; Goldhaber-Gordon et al. (2003) Biochemistry 42 14633-14642). The transposons used in the method of the invention surprisingly maintain a transposition efficiency similar to that of standard, unaltered mini-Mu.

The amino acid sequence may be altered by the deletion, insertion or substitution of at least one amino acid. Preferably, a single amino acid is deleted, inserted or substituted.

Where at least one amino acid is inserted into the amino acid sequence of the target polypeptide, or where at least one amino acid is deleted from the amino acid sequence of the target polypeptide, the method according to the first aspect of the invention preferably comprises the following steps:
a) conducting a transposition reaction comprising mixing the transposon, the target DNA and a transposase enzyme;
b) digestion of DNA resulting from (a) with a first restriction enzyme which recognises the first restriction enzyme recognition sequence contained in the transposon;
c) separation of DNA which does not comprise the transposon;
d) conducting an intramolecular ligation reaction of the DNA from (c); and
e) expression of protein from the DNA from (d).

For example, a host organism may be transformed with the DNA from (d), the protein then being expressed in the host organism. Alternatively, the protein may be expressed from the DNA from (d) using an artificial expression system, such as the Rapid Translation System available from Roche Diagnostics Ltd (Lewes, United Kingdom).

The skilled person will be aware of the options available for the expression of protein from DNA.

Where at least one amino acid of the amino acid sequence of the target polypeptide is substituted with a different amino acid, the method according to the first aspect of the invention preferably comprises the following steps:
a) conducting a transposition reaction comprising mixing the transposon, the target DNA and a transposase enzyme;
b) digestion of DNA resulting from (a) with a first restriction enzyme which recognises the first restriction enzyme recognition sequence contained in the transposon;
c) separation of DNA which does not comprise the transposon;
d) conducting an intermolecular ligation of DNA from (c) with a second DNA sequence comprising at least two second restriction enzyme recognition sites located such that at least one of the cleavage sites is not at a terminus of the second DNA sequence;
e) conducting the transformation of a host organism with DNA from (d) and selecting cells containing the second DNA sequence;
f) isolating DNA from cells selected in (e) and digestion of that DNA with a second restriction enzyme which recognises the second restriction enzyme recognition site, the second restriction enzyme being an outside cutter;
g) conducting an intramolecular ligation of DNA from (f); and
h) expression of protein from the DNA from (g).

For example, a host organism may be transformed with the DNA from (g), the protein then being expressed in the host organism. Alternatively, the protein may be expressed from the DNA from (g) using an artificial expression system, such as the Rapid Translation System available from Roche Diagnostics Ltd.

Preferably, step (f) above is followed by an additional separation step (f1), such that DNA which does not comprise the second DNA sequence is separated from DNA which does comprise the second DNA sequence. The DNA not comprising the second DNA sequence is then used in step (g).

In step (d) above, the phrase "cleavage site is not at a terminus of the second DNA sequence" indicates that the second restriction enzyme recognition site is located such that the cleavage site is one or more nucleotides from a terminus of the second DNA sequence, i.e. the cleavage site is within the second DNA sequence. The skilled person will readily appreciate the location of the second restriction enzyme recognition site which is required in order to gain a desired result of one or more amino acids being substituted.

The second restriction enzyme may be the same as the first restriction enzyme. Preferably, the second DNA sequence comprises a gene which gives a host cell containing the second DNA sequence a selectable characteristic compared to a cell not containing the second DNA sequence. The term "selectable characteristic", as used throughout this specification, may indicate, for example (where the cell is a bacterium), the ability to grow on an antibiotic-containing medium.

Where the amino acid sequence of the target polypeptide is altered by the insertion of a further amino acid sequence, the method according to the first aspect of the invention preferably comprises the following steps:
a) conducting a transposition reaction comprising mixing the transposon, the target DNA and a transposase enzyme;
b) digestion of DNA resulting from (a) with a first restriction enzyme which recognises the first restriction enzyme recognition sequence contained in the transposon;
c) separation of DNA which does not comprise the transposon;
d) conducting an intermolecular ligation of DNA from (c) with a third DNA sequence encoding for a further amino acid sequence; and
e) expression of protein from the DNA from (d).

For example, a host organism may be transformed with the DNA from (d), the protein then being expressed in the host organism. Alternatively, the protein may be expressed from the DNA from (d) using an artificial expression system, such as the Rapid Translation System available from Roche Diagnostics Ltd.

The further amino acid sequence may be a full protein, a protein domain or a protein fragment. The protein fragment may be (but is not limited to) an epitope, a binding domain, an allosteric site, a defined functional region such as a metal binding site, or an oligomerisation interface. Preferably, the third DNA sequence comprises a gene which gives a host cell containing the third DNA sequence a selectable characteristic compared to a cell not containing the third DNA sequence.

The third DNA sequence may have an open reading frame which is the same as that of the target DNA, so that when the DNA is translated into a protein, a single chimeric protein is created. Alternatively or additionally, the third DNA sequence may contain a stop codon and/or an initiation codon.

In a preferred embodiment of the method according to the invention, the first restriction enzyme is a Type IIS enzyme and, most preferably, is *Mly*I.

Preferably, the transposon has a low target site preference. The transposon may be derived from one of: mini-Mu, AT-2 or Tn5. The transposon preferably comprises a gene which gives a host cell containing the transposon a selectable characteristic compared to a cell not containing the transposon. More preferably, the transposon comprises the DNA sequence 5'-NGACTC-3' (SEQ ID NO:1) as the 5' terminal and 5'-GAGTCN-3' (SEQ ID NO:2) as the 3' terminal (preferably 5'-TGACTCGGCGCA-3' (SEQ ID NO:3) as the 5' terminal and 5'-TGCGCCGAGTCA-3' (SEQ ID NO:4) as the 3' terminal), or alternatively comprises the DNA sequence 5'-NNNNGACTC-3' (SEQ ID NO:5) as the 5' terminal and 5'-GAGTCNNNN-3' (SEQ ID NO:6) as the 3' terminal (preferably 5'-TGAAGACTCGCA-3' (SEQ ID NO:7) as the 5' terminal and 5'-TGCGAGTCTTCA-3' (SEQ ID NO:8) as the 3' terminal), where N is any nucleotide. In another alternative, the transposon comprises the DNA sequence 5'-TGTTGACTC-3' (SEQ ID NO:9) as the 5' terminal and 5'-GAGTCAACA-3' (SEQ ID NO:10) as the 3' terminal, or in yet another alternative comprises the DNA sequence 5'-CTGACTC-3' (SEQ ID NO:11) as the 5' terminal and 5'-GAGTCAG-3' (SEQ ID NO:12) as the 3' terminal.

The target DNA may be carried in a construct such as a plasmid, preferably pNOM or a derivative thereof.

According to a second aspect of the invention, there is provided a transposon comprising a restriction enzyme recognition sequence towards each of its termini, the recognition sequence being recognised by a restriction enzyme which is an outside cutter, the recognition sequence not being present in the remainder of the transposon and being positioned such that the restriction enzyme has a DNA cleavage site positioned beyond the end of the terminus of the transposon. Preferably, each restriction enzyme recognition sequence is positioned one or more nucleotides from a terminus of the transposon, more preferably between 1 and 20 nucleotides from a terminus of the transposon, yet more preferably between 1 and 10 nucleotides from a terminus of the transposon and most preferably 1, 2, 3, 4 or 5 nucleotides from a terminus of the transposon. Advantageously, this allows the transposon to be used as a tool in a method according to a first aspect of the invention, allowing the investigation of the impact of insertions, deletions and substitutions of one or more amino acids at points throughout a polypeptide of interest.

Surprisingly, the inventor has found that it is possible to engineer a transposon to be suitable for use in a method according to the first aspect of the invention, by altering the termini of the transposon without disrupting the ability of a transposase enzyme to recognise the transposon. For example, it was previously shown that mutations which change the termini of mini-Mu can have an adverse effect on the ability of MuA transposase to recognise the transposon (Goldhaber-Gordon et al. (2002) J. Biol. Chem. 277 7703-7712; Goldhaber-Gordon et al. (2003) Biochemistry 42 14633-14642). The transposons used in the method of the invention surprisingly maintain a transposition efficiency similar to that of standard, unaltered mini-Mu.

In a preferred embodiment, the restriction enzyme is a Type IIS enzyme and, most preferably, is *Mly*I*.* The transposon may comprise the DNA sequence 5'-NGACTC-3' as the 5' terminal and 5'-GAGTCN-3' as the 3' terminal (preferably 5'-TGACTCGGCGCA-3' as the 5' terminal and 5'-TGCGCCGAGTCA-3' as the 3' terminal). Alternatively, the transposon may comprise the DNA sequence 5'-NNNNGACTC-3' as the 5' terminal and 5'-GAGTCNNNN-3' as the 3' terminal (preferably 5'-TGAAGACTCGCA-3' as the 5' terminal and 5'-TGCGAGTCTTCA-3' as the 3' terminal). In a further alternative, the transposon may comprise the DNA sequence 5'-TGTTGACTC-3' as the 5' terminal and 5'-GAGTCAACA-3' as the 3' terminal. In another alternative, the transposon may comprise the DNA sequence 5'-CTGACTC-3' as the 5' terminal and 5'-GAGTCAG-3' as the 3' terminal. These termini sequences may include variations provided that the transposon remains viable for transposition and that the restriction enzyme recognition sites are at the required positions.

For example, the mini-Mu transposon may be modified close to both its termini to incorporate the recognition sequences for the type IIS restriction enzyme *Mly*I. The mini-Mu transposon includes the Cam^{R} gene which allows *E. coli* cells containing the transposon to grow in the presence of chloramphenicol. The skilled person will understand that recognition sequences for restriction enzymes other than *Mly*I may be introduced into the transposon, providing that the appropriate routine modifications are made to the methods described herein and extra steps added if required. Such modifications are routine to the skilled person.

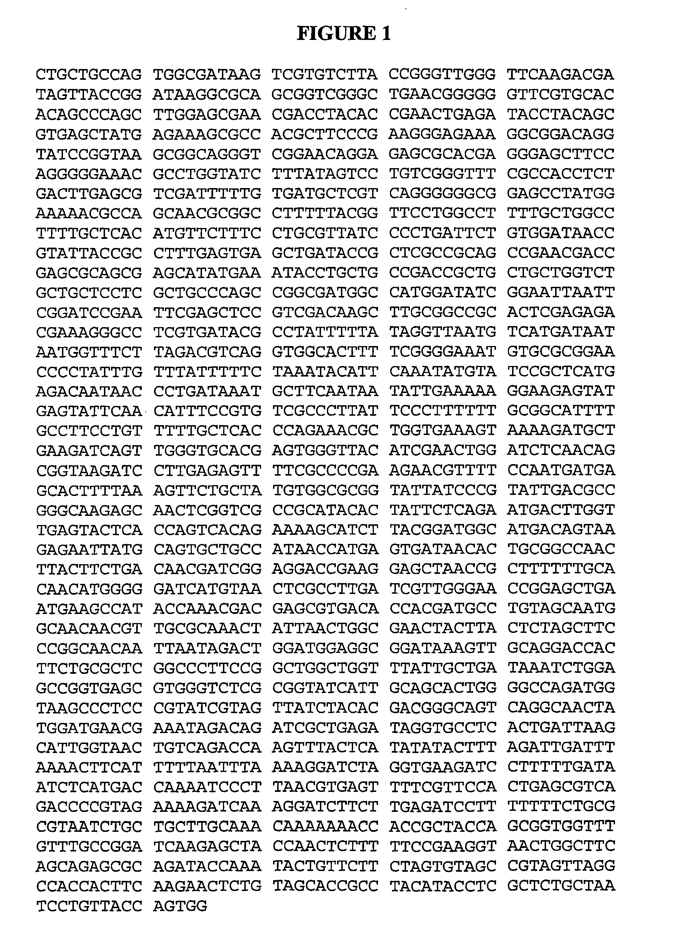

According to a third aspect of the invention, there is provided a plasmid having the DNA sequence shown in Figure 1, or a derivative of a plasmid having the DNA sequence shown in Figure 1. The term "a derivative of a plasmid having the DNA sequence shown in Figure 1" means a plasmid which has been adapted from the plasmid shown in Figure 1, for example by silent mutations in the DNA sequence, substitution of the *bla* gene with an alternative selectable marker, or by alteration of non-essential elements of the DNA sequence, such as sequences which do not form one of the essential elements of the plasmid such as the *ori* regions, the Multiple Cloning Site, or the *bla* gene. The term also includes the DNA sequence shown in Figure 1 with an additional DNA sequence of interest inserted at a point in the DNA sequence of Figure 1, preferably (but optionally) at the Multiple Cloning Site. The DNA sequence of a derivative of a plasmid having the DNA sequence shown in Figure 1 does not comprise the recognition sequence for the first restriction enzyme to be used in the method according to the first aspect of the invention, the derivative being intended for use in that method. The term "a derivative of a plasmid having the DNA sequence shown in Figure 1" is not intended to encompass the pUC18 plasmid.

According to a fourth aspect of the invention, there is provided a kit comprising a transposon according to a second aspect of the invention. Preferably, the kit further comprises a plasmid according to the third aspect of the invention. The kit may yet further comprise a suitable transposase and/or buffers required for the enzymatic reactions and/or oligonucleotides suitable for use in screening and/or DNA sequencing procedures. Most preferably, the kit is for use in the method according to the first aspect of the invention.

According to a fifth aspect of the invention, there is provided a method of determining whether the introduction of a mutation into a target polypeptide alters a detectable activity of that polypeptide, comprising the method according to the first aspect of the invention and the further steps of:
a) screening for a difference in the activity of the altered target polypeptide compared to the unaltered target polypeptide; and
b) sequencing the altered target polypeptide to determine the location of the amino acid insertion, deletion or substitution.

Examples of a detectable activity include, where the protein is an enzyme, substrate binding activity; where the protein is an antibody, antigen binding activity; where the protein is a receptor, ligand binding activity. The skilled person will readily understand means by which the activity of other protein types can be assessed.

### BRIEF DESCRIPTION OF THE DRAWINGS

Embodiments of the invention will now be described, by way of example only, with reference to Figures 1-10 in which:
Figure 1 shows the DNA sequence of pNOM (SEQ ID NO: 1 00);
Figure 2 shows an outline of the triplet nucleotide deletion-insertion mutagenesis method;
Figure 3A shows the sequences of the engineered MuDel and MuIns transposon termini, Figure 3B shows the mechanism for the introduction of a three nucleotide base pair deletion and Figure 3C shows the mechanism for the introduction of a three nucleotide base pair insertion;
Figure 4 shows an analysis of library BLA^{DEL} with *Mly*I to determine the randomness of transposon insertion: (A) shows an illustration of the restriction analysis procedure; (B) shows the restriction analysis of 15 of the 22 BLA^{DEL} library members (the band labelled with an asterisk corresponds to the transposon); and (C) shows the position of the transposon insertion points in the 22 members of the BLA^{DEL} library as determined by DNA sequencing;
Figure 5 shows the determination of ampicillin MIC values for each selected member of library BLA^{DEL};
Figure 6 shows the outline of the triplet nucleotide substitution mutagenesis method;
Figure 7A shows the basic features of the SubSeq DNA element for substitution mutagenesis and Figure 7B shows the mechanism for the introduction of a three nucleotide base pair substitution;
Figure 8 shows the outline of the creation of a library of variants containing insertions of whole proteins, protein domains or fragments (such as epitopes) of protein domains;
Figure 9A shows the features of the AT-2 based transposon suitable as an alternative to MuIns and Figure 9B shows the mechanism by which the modified AT-2 transposon can be used to create a library of target genes with triplet nucleotide insertions; and
Figure 10A shows the features of the Tn5InsOE and Tn5InsME transposons suitable as an alternative to MuIns and Figure 10B shows the mechanism by which the Tn5InsOE and Tn5InsME transposons can be used to create a library of target gene with triplet nucleotide insertions.

### MODES OF CARRYING OUT THE INVENTION

### Materials

Bacterial strains: *Escherichia coli* DH5α (*supE*44, Δ*lacU*169, (φ80 *lacZ*ΔM15)*, hsdR*17, *recA*1, *endA*1, *gyrA*96, *thi*-1, *relA*1).

Plasmids: pUC18, pEntranceposon (*Cam*^{r}) (Finnzymes, Esboo, Finland) and pNOM.

Transposons: The transposons used for insertion-deletion mutagenesis are based on mini-Mu (Cam^{R}-3).

Antibiotics: Ampicillin and chloramphenicol (both Melford Laboratories, Ipswich, UK).

DNA-related enzymes: *Taq* DNA polymerase (Promega Corp., Madison, WI, USA), Extensor Hi-Fidelity PCR enzyme (Abgene, Epsom, UK), *Mly*I*, Xho*I*, Bgl*II and *Nde*I restriction endonucleases (NE Biolabs, Beverly, MA, USA), T4 DNA ligase (Abgene), MuA transposase (Finnzymes), EZ-Tn5™ transposase (Epicentre, Madison, WI, USA).

Genes: *Bla* gene encoding TEM-1 β-lactamase.

DNA purification kits: The isolation of plasmid DNA from cell cultures was performed using the Wizard^{®} Plus SV kit from Promega Corp. DNA was isolated from agarose gel or PCR reactions using the Qiaquick^{™} Gel extraction or PCR purification kits, respectively, supplied by Qiagen Ltd, Crawley, UK.

### Methods and Results

### Example 1

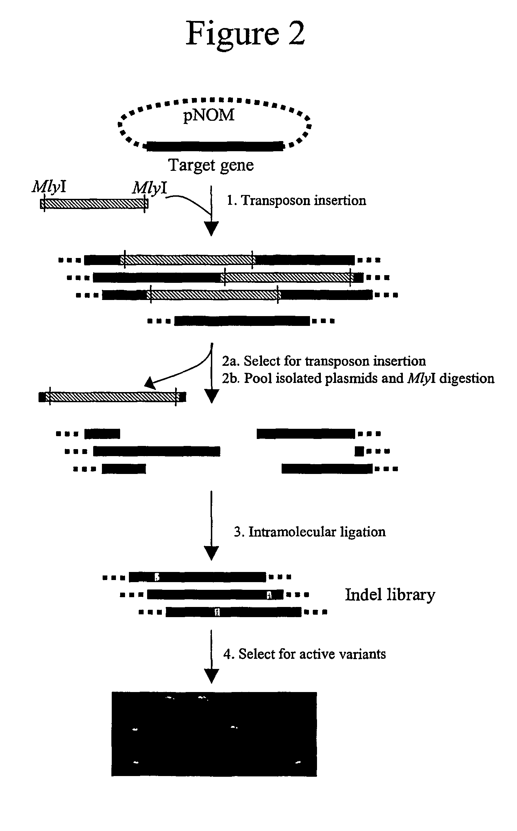

This example illustrates how to create a library of variants containing triplet nucleotide deletions at random positions in the *bla* gene, as shown in Figure 2. In summary, the procedure consists of 4 main steps:
Step 1: The MuDel transposon is inserted into the target plasmid or target gene.
Step 2: Cells containing a plasmid-integrated MuDel contain the *Cam*^{R} gene and so can grow in the presence of chloramphenicol. The plasmids are isolated and pooled, and the transposon is removed by *Mly*I digestion.
Step 3: Intramolecular ligation results in the reformation of the target gene, minus nucleotide base pairs.
Step 4: The resulting library is subjected to a selection or screen to select those variants with the required properties.
In Figure 2, hatched blocks represent the transposon, solid blocks the *bla* gene, gaps the deletion point (for the purposes of this Example), grey blocks the deletion point (for the purposes of this Example) in the re-ligated target gene and the thick dashed lines the rest of the plasmid backbone.

The procedure can also be applied to a target gene other than the *bla* gene, provided that:
1. there are suitable modifications to the selection or screening step at step 4 in Figure 2 that are suitable to the protein encoded by the target gene; and
2. any undesirable restriction sites are either not present or removed from the target gene.

Describing this example now in detail, a modified mini-Mu transposon and a newly constructed pNOM plasmid are used. In this example, the restriction endonuclease *Mly*I is critical to triplet nucleotide deletion but other restriction endonucleases with properties similar to that of *Mly*I can be used, providing the appropriate steps are modified and extra steps added if required, as will be understood by the skilled person.

For the procedure to work, the target DNA or plasmid containing the target DNA must not contain any *Mly*I restriction sites. The recognition sequence of *Mly*I is only 5 bp in length, so many plasmids have at least one if not more *Mly*I restriction sites. For example, pUC 18 has four *Mly*I restriction sites, including one in the *bla* gene, one in the pMB1 origin of replication (*ori*) and another in the multiple cloning site (MCS).

### Construction of pNOM

Therefore, a suitable plasmid was constructed that contained no *Mly*I sites and a useful MCS, this new vector being called pNOM. The majority of the plasmid was donated by pUC18, including the *ori* regions and *bla* gene. The *Mly*I sites present in the *bla* gene were removed by the introduction of a silent mutation so as not to disrupt the primary structure of the TEM-1 β-lactamase. Removal of the *Mly*I site from the *ori* region was achieved by creating a library in which two of the nucleotides that form the *Mly*I recognition sequences were randomised, as it was unknown how rational mutations may affect plasmid replication. The MCS site was constructed to contain useful cloning sites.

Unless otherwise stated, all PCR reactions were performed with the Extensor Hi-Fidelity PCR Enzyme mix and its supplied buffers (Abgene). The pNOM plasmid was constructed from pUC18 and an artificial MCS. The -1 to 1979 bp region of pUC18 was amplified by PCR in several stages, so as to remove any *Mly*I restriction sites from the DNA sequences. The PCR reaction mixture was composed of 1 µl of 0.1 ng/µl of pUC18 as the template, 3 µl of 10 µM of suitable primer (see below for primer combinations), 3 µl of 20mM dNTP mixture (composed of 5mM dATP, 5mM dTTP, 5mM dGTP and 5mM dCTP), 5 µl of the 10x Extensor buffer 1, 0.5 µl of 5 Units/µl Extensor Hi-Fidelity PCR enzyme mix and made up to 50 µl with sterile molecular biology quality water. In each case, PCR was performed as shown below:
Step 1: 94°C for 2 min
Step 2: 94°C for 10s
Step 3: 55°C for 30s
Step 4: 68°C for 90s
Repeat steps 2 to 4 an additional 29 times
Step 5: 68°C for 7 min

Fragment F1 consisted of -1 to 989 bp of pUC18 and was produced by PCR using single stranded DNA in the form of chemically synthesised oligonucleotides (referred to as 'primers') DDJdi006 (5' GAAACtCGaGAGACGAAAGGGCCTCGTGATACG 3'; SEQ ID NO: 13) and DDJdi004 (5' CATCCATAGTTGCCTGACTgCCCGT CGTGTAGATAAC 3'; SEQ ID NO:14), with lower case letters signifying nucleotides undergoing mutagenesis; DDJdi006 introduced an *Xho*I site and DDJdi004 removed the *Mly*I site from the *bla* gene.

Fragment F2 consisted of 972 to 1507 bp of pUC18 and was produced by PCR using primers DDJdi003 (5' GTTATCTACACGACGGGcAGTCAGGCAACTATGGATG 3'; SEQ ID NO:15) and DDJdi008 (5' CCAACCCGGTAAGACAC 3'; SEQ ID NO:16). DDJdi003 is complementary to DDJdi004 and the lower case letter signifies nucleotides undergoing mutagenesis.

Fragment F3 consisted of 1490 to 1979 bp of pUC108 and was produced by PCR using primers DDJdi007 (5' GTGTCTTACCGGGTTGGNNTCAAGACGATAGTT ACCGGA 3'; SEQ ID NO:17) and DDJdi009 (5' cttcctcgctcatatgCTCGCTGC GCTCGGTCGTTCGGCTGC 3'; SEQ ID NO:18). DDJdi007 contained two randomised nucleotides (i.e. any nucleotide at each position indicated as "N") corresponding to the *Mly*I site in the pMB1 *Ori* origin of replication region of pUC18. DDJdi009 contained a *Nde*I recognition site towards its 5' end.

Fragments F1, F2, and F3 were isolated and purified after agarose gel electrophoresis. Each of the fragments was spliced together in a single PCR reaction using DDJdi006 and DDJdi009 as the terminal primers to create fragment F4. The extension temperature at 68°C was increased to 120 s in this PCR reaction. The 2005 bp product was isolated and purified after agarose gel electrophoresis. The fragment F4 was digested with *NdeI* and *XhoI* endonuclease under the recommended conditions (50 mM NaCl, 10 mM Tris-HCl (pH 7.9), 10 mM MgCl₂, 1 mM dithiothreitol (DTT) in the presence of 0.1 mg/ml bovine serum albumin (BSA)) and the DNA was purified from the restriction digestion mixture using the Qiagen Qiaquick^{™} PCR Purification kit.

Fragment F5 contained the new multiple cloning site (MCS) and is based on the MCS of pET22b. It was produced by PCR using primers pET-F (5' ATGCGTCCGGCGTAGAGGA 3'; SEQ ID NO:19) and pET-R (5' GCTAG TTATTGCTCAGCGGTG 3'; SEQ ID NO:20) and pET24b as the template, using standard PCR conditions except that the extension time at 68°C was 60 s. The resulting 351 bp product was purified and was digested with *Nde*I and *Xho*I followed by isolation and purification after gel electrophoresis.

The *Nde*I/*Xho*I digested F4 and F5 fragments were ligated together at 25°C using 1 µl of 3 U/µl T4 DNA ligase (Promega Corp.) under the conditions recommended by the manufacturer (30 mM Tris-HCL (pH7.8), 10 mM MgCl₂, 10 mM DTT and 1 mM ATP) and ¹/₁₀ of the ligation mixture was used to transform 50 µl of *E. coli* DHSα by electroporation using a Biorad Gene Pulser^{™} (Bio-Rad Laboratories, Hemel Hempstead, UK). 500 µl of SOC medium was added to the cells immediately after electroporation and the cells were incubated at 37°C for 1 hr. Approximately 50 and 500 µl of the recovering electroporated *E. coli* DH5α cells were spread on LB agar plates containing 100 µg/ml ampicillin and incubated at 37°C overnight. The correct ligation of fragments F4 and F5 represent the creation of the pNOM plasmid.

Five individual *E. coli* DH5α colonies capable of growth in the presence of 100 µg/ml ampicillin were picked and transferred to 5 ml of LB broth containing 100 µg/ml ampicillin and the cultures incubated at 37°C overnight in a rotary shaker. The plasmid DNA from three of the five cultures was purified. The plasmid DNA was subjected to restriction analysis with either *Nde*I or *Mly*I to confirm the nature of the plasmid and that all the *Mly*I sites had been removed. One clone was selected to act as the source of pNOM and DDJdi003 and DDJdi009 were used to amplify the *Ori* region by PCR to confirm the mutations due to the NN nucleotides in DDJdi007. Sequencing of this region was not possible but restriction analysis with *Mly*I reconfirmed that this region did not have a *Mly*I site. The DNA sequence of pNOM is shown in Figure 1.

### Construction of MuDel

The original Mu phage-derived transposon, mini-Mu (*Cam*^{R}-3)*,* was engineered for use in the creation of random triplet nucleotide deletions. In this case, the *Cam*^{R} gene is used as a selectable marker within the transposon. This can be exchanged for another gene that will provide a chosen strain of *E. coli* or any other suitable organism with a selection advantage under a particular condition so making the organism viable or displaying a characteristic that will differentiate it from other cells that do not contain the transposon sequence.

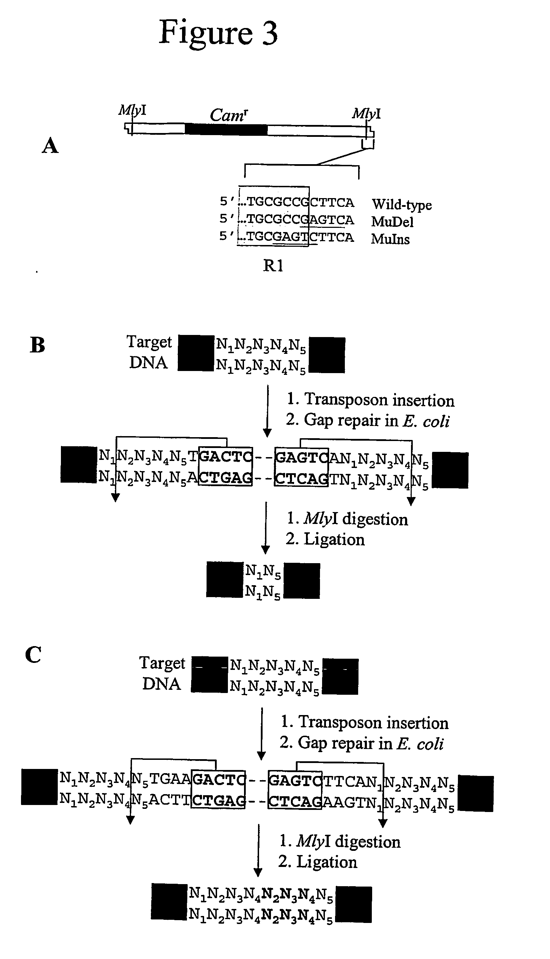

The ability to delete nucleotide triplets depends on the transposon insertion mechanism and the position of two introduced restriction sites, as outlined in Figure 3. The mini-Mu transposon was engineered so as to act as a vehicle for the insertion of specific restriction sites into the target gene (Figure 3A). The restriction endonuclease chosen was *Mly*I, a type IIS enzyme that cuts 5 bp outside its recognition sequence to generate a blunt end (cleavage profile 5' GAGTC(N₅)↓ 3'). The *Mly*I recognition site is to be placed 1 bp away from the site of transposon insertion, so creating MuDel (Figure 3A). The two required point mutations both lie outside the R1 region that is involved in MuA binding, so minimising disruption to the protein-DNA interactions that can potentially affect the efficiency of the transposition reaction. Transposition of MuDel will occur via a 5 bp staggered cut in the target DNA that, following *E. coli* gap repair, results in the duplication of these 5 bp (Figure 3B). Digestion of the DNA with *Mly*I removes the transposon along with four additional nucleotide base pairs from the target gene at both termini. Intramolecular ligation of the two blunt ends results in the in-frame deletion of 3 nucleotides from the target gene (Figure 3B).

Unless otherwise stated, all PCR reactions were performed with the Extensor Hi-Fidelity PCR Enzyme mix and performed as described above. The MuDel transposon was constructed by PCR using the oligonucleotide DDJdi005 (5' GCTTAGATCTGActCGGCGCACGAAAAACGCGAAAG 3' (SEQ ID NO:21); lower case letters signify nucleotides undergoing mutagenesis) as both the forward and reverse primer with 0.1 ng of the original mini-Mu (*Cam*^{R}-3) transposon acting as template. The 1322 bp product was purified and digested with *Bgl*II at 37°C (reaction conditions: 100 mM NaCl, 50 mM Tris HCl (pH 7.9), 10 mM MgCl₂, 1 mM DTT). The digested transposon was isolated and purified after agarose gel electrophoresis. The DNA representing the new transposon MuDel was recloned by ligation into *Bgl*II digested pEntranceposon (*Cam*^{R}) using T4 DNA ligase, and ¹/₁₀ of the ligation mixture was used to transform 50 µl of *E. coli* DH5α by electroporation using a Biorad Gene Pulser^{™}. 500 µl of SOC medium was added to the cells immediately after electroporation and the cells were incubated at 37°C for 1 hr. Approximately 50 and 500 µl of the recovering electroporated *E. coli* DH5α cells were spread on LB agar plates containing 20 µg/ml chloramphenicol and incubated at 37°C overnight. Six individual *E. coli* DH5α colonies capable of growth in the presence of 20 µg/ml chloramphenicol were replica plated on another LB agar plate containing 20 µg/ml chloramphenicol. Part of the original colony was used as the source of template DNA in a PCR reaction using *Taq* DNA polymerase as the thermostable enzyme, and pUC-F (5' AGCTGGCGAAAGGGGGATGTG 3'; SEQ ID NO:22) and pUC-R (5' TTATGCTTCCGGCTCGTATGTTGTGT 3'; SEQ ID NO:23) as the primers. PCR was performed using the conditions stated above for *Taq* DNA polymerase. The PCR mixture contained 5 µl of 10x reaction buffer (100 mM Tris-HCl (pH 9.0), 500 mM KCL, 1% Triton X-100), 3 µl 25 mM MgCl₂, 3 µl 20 mM dNTPs, 1.5 µl 10 µM oligonucleotide primer, an appropriate *E. coli* colony and 0.5 µl of 5 U/µl *Taq* DNA polymerase. The reaction mixtures were made up to 50 µl with molecular biology quality water. The reaction mixtures were subjected to the following thermocycling conditions:
Step 1: 94°C for 3 min followed by addition of 0.5 µl *Taq* DNA polymerase
Step 2: 94°C for 20s
Step 3: 55°C for 20s
Step 4: 72°C for 90s
Steps 2 to 4 repeated an additional 29 times
Step 5: 72°C for 5 min.

The 1504 bp product was purified and digested with *Mly*I at 37°C (reaction conditions: 50 mM potassium acetate, 20mM Tris-acetate, 10mM magnesium acetate, 1mM DTT (pH 7.9), supplemented with 100 µg/ml BSA) and analysed by agarose gel electrophoresis to confirm the presence of the *Mly*I recognition sequence. The colonies containing the MuDel transposon were transferred to 5 ml of LB broth containing 100 µg/ml ampicillin and the cultures incubated at 37°C overnight in a rotary shaker and the plasmid DNA was purified. The plasmid DNA was sequenced using primers pUC-F and pUC-R to confirm the sequence of MuDel. The MuDel transposon was released from the context of the plasmid by digestion with *Bgl*II under the conditions stated above and purified after agarose gel electrophoresis.

### Transposition reaction and transformation into E. coli cells

Transposition with mini-Mu and the MuDel transposon was performed at 30°C for 3 hr followed by heat inactivation at 75°C for 10 min. The reaction mixture was composed of 2 µl of reaction buffer (125 mM Tris-HCl, pH 8.0, 125 mM MgCl₂, 50 mM NaCl, 0.25% Triton X-100 and 50% (v/v) glycerol), 1 µl of 0.22 µg/ml MuA transposase and varying quantities of target DNA and transposon as quoted below. The efficiency of the transposition reaction using MuDel was tested using the control DNA template supplied by Finnzymes (pUC19 containing a 6.6 kbp *Hind*III fragment of bacteriophage λ DNA cloned into the *Hind*III site) and pUC18. The pNOM plasmid was used in the construction of libraries. Either 360 ng (control DNA) or 100 ng (pUC18 or pNOM) of target plasmid DNA and either mini-Mu (Cam^{R}-3) (20 ng) or MuDel (20 ng or 100 ng) were present in the reaction mixture. The reactions were left at 30°C for 3 hr followed by heat inactivation at 75°C for 10 min. Either 1 µl or 2 µl were used to transform *E. coli* DH5α cells by electroporation and the cells were plated on LB agar containing 20 µg/ml chloramphenicol to select for cells containing the *Cam*^{R} gene and hence the mini-Mu or MuDel transposon.

To test if the introduced mutations disrupted transposition efficiency, pUC18 was used as the target DNA substrate. The transposition reaction with mini-Mu transposon (20 ng) resulted in the growth of approximately 99 *E. coli* DH5α colonies on 20 µg/ml chloramphenicol plates after transformation by electroporation with ¹/₁₀ (2 µl) the transposition reaction mixture. Replacing the mini-Mu transposon with either 20 ng or 100 ng of MuDel resulted in the growth of approximately 100 and 430 colonies, respectively. Surprisingly, therefore, MuDel still acts as an efficient substrate for the transposition reaction, despite the introduction of mutations at the termini of the transposon.

As mentioned, the general outline of the method for the creation of triplet nucleotide deletions at random positions within a target gene is shown in Figure 2. The *bla* gene that encodes TEM-1 β-lactamase was chosen as the target as it is a clinically important enzyme responsible for resistance to some β-lactam antibiotics and mutagenesis of the enzyme can lead to resistance to new ES β-lactams. It also provides an easy selection method, as active variants will confer resistance to ampicillin on *E. coli* so permitting cell growth. The new vector, pNOM, was used as the source of the *bla* gene and therefore acts as the target DNA for MuDel insertion

As an alternative to the above description, the gene of interest independent of pNOM can be used as the target for transposon insertion. If required, the gene of interest can be cloned into pNOM or another suitable vector using standard techniques after transposon insertion. Alternatively, after transposon insertion into the gene of interest, the gaps present in the DNA strands formed as a result of the transposition reaction that are normally repaired in the organism can be repaired *in vitro* using the appropriate gap repair and ligation techniques.

The place of insertion of MuDel into pNOM should be distributed evenly throughout the plasmid and so a strategy is required that will select for cells containing MuDel inserted into the *bla* gene region. The transposition of the MuDel transposon into the plasmid DNA confers resistance to chloramphenicol on *E. coli,* allowing for selection of cells containing MuDel-inserted pNOM. Those colonies that have MuDel inserted within the *bla* gene region will disrupt TEM-1 expression and thus affect the cells' ability to grow in the presence of ampicillin.

### Selection of colonies with transposon-disrupted bla gene

After transformation of *E. coli* DH5α with the transposition mixture, 48 colonies were selected that grew on 20 µg/ml chloramphenicol and replated on both a 100 µg/ml ampicillin and a 20 µg/ml chloramphenicol LB agar plates. Of the 48 colonies, 22 grew only on the chloramphenicol plate and were deemed to have a disrupted *bla* gene due to transposon insertion in this region and therefore chosen as the members of the BLA^{DEL} library. To confirm the presence of the MuDel transposon, PCR was performed on each of the 22 colonies using *Taq* DNA polymerase (method described above) and primers DDJdi010 (5' TCCGCTCATGAGACAATAACCCTG 3'; SEQ ID NO:24) and DDJdi011 (5' CTACGGGGTCTGACGCTCAGTG 3'; SEQ ID NO:25) that flank the *bla* gene.

### Restriction analysis and selection of clones including inserted transposon

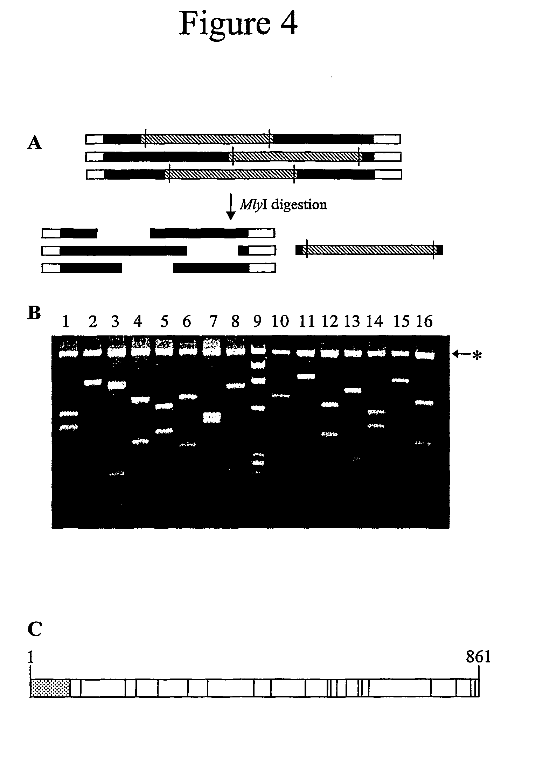

The PCR products were purified and restriction analysis was performed with *Mly*I (reaction conditions described previously) to confirm the diversity of transposon insertion positions (Figure 4). Digestion of the linear PCR fragment (containing only the *bla* gene regions of pNOM) with *Mly*I results in the removal of the MuDel transposon and 8 bp of the *bla* gene (1310 bp), generating two fragments of varying length, depending on the MuDel insertion point (Figure 4A). The restriction analysis revealed that the insertion of MuDel occurred randomly and only one transposon was inserted in this region (Figure 4B - lanes 1 to 8 and 10 to 16 represent different members of the BLA^{DEL} library and lane 9 is the φ174 DNA-*Hae*III molecular weight ladder. The band labelled with an asterisk corresponds to the transposon). Mass analysis of the two smaller fragments from each lane confirmed that the cumulative size of the two fragments was approximately equal to that of the PCR product minus MuDel. The 22 PCR products were sequenced using DDJdi010 and DDJdi011 as the primers to determine the position of the transposon within the *bla* gene. Sequence analysis confirmed the restriction analysis that the transposon insertion occurred at random positions within the *bla* gene, indicated by vertical lines in Figure 4C.

The MuDel-inserted pNOM plasmids were isolated from each of the 22 colonies and equal amounts of each plasmid were pooled and subjected to restriction digestion with *Mly*I followed by agarose gel electrophoresis. The band corresponding to the linear pNOM minus MuDel was isolated and purified after agarose gel electrophoresis. Intramolecular ligation was performed using T4 DNA ligase and approximately 10 ng of linear pNOM (reaction conditions described above). The reaction was left at 25°C for 10 min followed by 10 hr at 16°C. *E. coli* DH5α cells were transformed by electroporation with 1 µl of the ligation mixture. 500 µl of SOC medium was added to the cells immediately after electroporation and the cells were incubated at 37°C for 1 hr. 50 µl and 500 µl of the recovering transformed cell cultures were plated on LB agar plates containing 15 µg/ml ampicillin. The plates were left overnight at 37°C and 94 BLA^{DEL} library and two pNOM-containing colonies were selected and transferred to 96 deep-well culture plates containing 200 µl LB medium and 15 µg/ml ampicillin. The cells were grown for 16 hr at 37°C with vigorous shaking. Sterile glycerol was added to 10% (v/v) for storage at -80°C.

### Effect of mutations on TEM-1 β-lactamase activity

The TEM-1 β-lactamase activity of each colony was measured *in vivo* by determining the minimum inhibitory concentration (MIC) of ampicillin that prevents *E. coli* growth. Each colony in the 96 well plates was replica plated on LB agar in Nunc Omnitray^{™} plates containing 50, 100, 500, 2500, 5000, 7500 or 10000 µg/ml Amp using a 96 prong replication fork and incubated at 37°C for 16 hr.

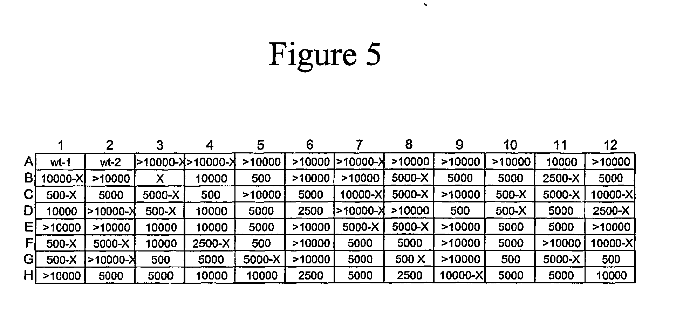

The MIC of each original colony for ampicillin is shown in Figure 5, showing the determination of ampicillin MIC values for each selected member of library BLA^{DEL}, with the 96 well microplate format used to label rows (A-H) and columns (1-12). The values in the boxes represent ampicillin MIC values of 500, 2500, 5000 and 10,000 µg/ml or >10,000 µg/ml. Boxes marked with an X following the number indicates variants with *bla* gene sequence information. All the cells grew at both 50 and 100 µg/ml ampicillin. Fourteen had a MIC of 500 µg/ml indicating reduced TEM-1 activity. Only six had a MIC of 2500 µg/ml and 30 had a MIC of 5000 µg/ml. No variants had a MIC at 7500 µg/ml ampicillin and the growth of 15 variants was inhibited at 10000 µg/ml Amp. The remaining 31 colonies were still viable at 10000 µg/ml Amp, including the two wild-type pNOM controls. Such a spread of MIC values indicates that various 3 nucleotide base pair deletion mutations have been incorporated into the *bla* gene and have had a profound effect on the *in vivo* activity of TEM-1.

Several clones that exhibited MIC at each Amp concentration were subjected to PCR with primers DDJdi010 and DDJdi011 and *Taq* DNA polymerase. The 1067 bp PCR products were sequenced with DDJdi010 and DDJdi011 as sequencing primers to confirm the position of triplet nucleotide deletion.

The point of insertion of MuDel with respect to a single codon will determine the nature of the deletion. The three possibilities are shown in columns 1, 4 and 5 of Table 1. One third of all insertions will create a true deletion of a codon. In the other two thirds, the 3 nucleotide base pairs removed will overlap two codons that may result in a secondary point mutation. The nature of the secondary mutation will vary depending on the surrounding DNA sequence. Due to the degeneracy of the genetic code, some of the point mutations will be silent while others will result in amino acid substitutions.

**Table 1. The potential outcomes with respect to the insertion of MuDel at the three different positions within a codon (columns 4 & 5) and insertion of MuIns at the three different positions within a codon (columns 2 & 3 - see Example 2 below).**

| **1** | **2** | **3** | **4** | **5** |
|---|---|---|---|---|
| **Transposon insertion (↓)** | **Triplet nucleotide insertion** | **Protein sequence** | **Triplet nucleotide deletion** | **Protein sequence** |
| GGG TTT CCC | GGG TTT CCC | Gly-Phe-Pro | GGG TTT CCC | Gly-Phe-Pro |
| GGG TTT C↓CC | GGG TTT TTT CCC | Gly-Phe-Phe-Pro | GGG CCC | Gly Pro |
| GGG TTT CC↓C | GGG TTT CTT CCC | Gly-Phe-Leu-Pro | GGG T CC | Gly Ser |
| GGG TTT CCC↓ | GGG TTT CCT CCC | Gly-Phe-Pro-Pro | GGG TT C | Gly Phe |

Several *bla* genes were isolated from clones exhibiting specific ampicillin MICs and sequenced to confirm the position of the amino acid deletion and if any secondary mutations have occurred. Table 2 shows all the different sequences isolated from active TEM-1 variants of library BLA^{DEL}_{.}

**Table 2. The determined 3 base pair deletions. The 5 bp duplicated in during transposon insertion are shown in bold. The amino acid sequences are numbered using the recommended numbering systems (Ambler, R.P. et al. (1991) Biochem J. 276 (Pt 1) 269-270). The new codons generated after deletion are underlined. Δ after an amino acid residue number signifies that the residue has been deleted.**

| **Wild-type sequence** | **Amino acid sequence** | **Deletion Sequence** | **Mutation** |
|---|---|---|---|
| CGCCCCGAAGAA | 61-RPEE-64 | CGCC---AAGAA | P62Δ-E63Q |
| TTATCCCGTATT | 81-LSRI-84 | TTATCC---ATT | R83Δ |
| CATCTTACGGAT | 112-HLTD-115 | CATCT---GGAT | T114Δ |
| CATCTTACGGAT | 112-HLTD-115 | CATCTTA---AT | T114Δ-D115N |
| GACGAGCGTGAC | 176-DERD-179 | GACGA---TGAC | E1770-R178D |
| TTAACTGGCGAA | 194-LTGE-197 | TTAAC---CGAA | G196Δ |
| GTTGCAGGACCA | 216-VAGP-219 | GTTG---GACCA | A217Δ |
| GATGAACGAAAT | 273-DERN-276 | GATGAA---AAT | R275Δ |

Of the 22 potentially different mutations, 8 were identified under this selection criterion as being tolerated by TEM-1. Two of the eight (R83Δ and R275Δ; Δ denotes the residue deleted) were true codon deletions. Three of the sequences (T114Δ, G196Δ and A217Δ) did not generate true deletions at the genetic level but, due to the degeneracy of the genetic code, no amino acid substitutions resulted. Three of the sequences (P62Δ-E63Q, T114Δ-D115N and E177Δ-R178D) contained a secondary mutation. The MuDel insertion point with respect to a single codon is evenly distributed (2:3:3) as expected (Table 2). No other mutations were observed for any of the sequenced variants and no wild-type TEM-1 was detected.

The sequences were spread across the whole length of the primary structure of TEM-1 with varying affects on the *in vivo* activity. Two mutations, T114Δ and T114Δ-D115N were only separated by a transposon insertion position of 2 nucleotide base pairs (Table 2).

There was a good correlation between the sequences and the ampicillin MIC (Table 3). The P62Δ-E63Q, R83Δ and E177Δ-R176D containing variants all had a MIC of 500 µg/ml. The T114Δ and T114Δ-D115N variants spanned both the 2500 µg/ml and 5000 µg/ml values with multiple clones identified at each concentration. The R275Δ variant has a relatively high MIC for amp (5000 µg/ml) even though the deletion takes place within a helix. Both A217Δ and G196Δ have very little effect on *in vivo* activity, with the G196Δ still able confer resistance on *E. coli* to Amp at 10000 µg/ml. One clone with the G196Δ TEM-1 variant did exhibit a MIC of 10000 µg/ml ampicillin but it is unknown why, as the general trend for the G196Δ variant (6 out of 7 sequenced; Table 3) indicated that cells with this variant could grow at ampicillin concentrations of 10000 µg/ml.

**Table 3. The relationship between Amp MIC and the nature of the deletion mutation. No cell had a MIC at 100 or 7500 µg/ml. The frequency refers to the number of sequenced bla genes with that mutation at that particular Amp MIC value. The location of the mutation with regards to the secondary structure (see Jelsch, C., et al. (Proteins (1993) 16 364-383) for nomenclature) is also shown. The residues are numbered using the recommended numbering systems (Ambler, R.P. et al. (1991)).**

| **Amp MIC (µg/ml)** | **Mutations** | **Frequency** | **Secondary structure** |
|---|---|---|---|
| 100 | - | - | |
| 500 | P62Δ-E63Q | 3 | Loop S2-SB1 |
| | R83Δ | 2 | H2 |
| | E177Δ-R178D | 1 | Ω loop |
| 2500 | T114Δ | 1 | Loop H3-SC4 |
| | T114Δ-D115N | 2 | Loop H3-SC4 |
| 5000 | T114Δ | 2 | Loop H3-SC4 |
| | T114Δ-D115N | 2 | Loop H3-SC4 |
| | R275Δ | 5 | H11 |
| 7500 | - | - | |
| 10000 | G196Δ | 1 | Loop H8-H9 |
| | A217Δ | 5 | Loop H9-H10 |
| >10000 | G196Δ | 6 | Loop H8-H9 |
| | Wild-type (pNOM) | | |

### Example 2

This example illustrates how to create a library of variants containing triplet nucleotide insertions at random positions in the target gene of interest using the transposon-based technology as outlined in Example 1. This example uses a modified mini-Mu transposon and the newly constructed pNOM plasmid or a suitable derivative of the pNOM plasmid. In this example, the restriction endonuclease *Mly*I is critical to triplet nucleotide insertion but other restriction endonucleases with properties similar to that of *Mly*I can be used, providing the appropriate steps are modified and extra steps added if required, as will be understood by the skilled person.

This example follows the procedure outlined in Example 1 and Figure 2. In Figure 2, for the purposes of this Example, gaps and grey blocks represent the insertion point. The main difference is with respect to the mini-Mu transposon. The original Mu phage-derived transposon, mini-Mu (*Cam*^{R}-3)*,* is engineered for the creation of random triplet nucleotide insertions. In this case, the *Cam*^{R} gene is used as a selectable marker within the engineered Mu transposon. Providing the correct elements are present at the termini of the transposon to allow transposition, the *Cam*^{R} gene may be exchanged for another gene that will provide a chosen strain of *E. coli* or any other suitable organism with a selection advantage under a particular condition so making the organism viable or displaying a characteristic that will differentiate it from other cells that do not contain the transposon sequence.

The engineered transposon, known as MuIns, contains the *Mly*I restriction site but its position was shifted to 4 nucleotide base pairs away from the site of transposon insertion (Figure 3A). The mechanism for triplet nucleotide insertion follows a similar path to that in Example 1 and is outlined in Figure 3C. Transposon insertion results in a five bp duplication after gap repair in *E. coli* (the 4 bp overhang from the transposon is removed). The cleavage site of *Mly*I is 5 bp away from the recognition sequence resulting in the removal of 1 bp of the target gene at both ends. Ligation of the two termini rejoins the gene but with the addition of 3 nucleotide base pairs. The three nucleotide base pairs inserted are shown in bold in Figure 3C. The point of insertion of MuIns with respect to a single codon will determine the nature of the insertion. The three possibilities are shown in columns 1, 2 and 3 of Table 1 above.

The transposon was used in the same manner as with the MuDel and the same approach was taken: insertion of the transposon into the target gene; selection for the transposon-inserted target gene; removal of the transposon by restriction digestion; intramolecular ligation; transformation into a suitable organism; select or screen a library of target gene variants with a triplet nucleotide insertion.

### Example 3

This example illustrates how to create a library of variants containing triplet nucleotide substitutions at random positions in the target gene of interest using the transposon-based technology as outlined in Example 1. This example uses the MuDel transposon (as outlined in Example 1) and the newly constructed pNOM plasmid or a suitable derivative of the pNOM plasmid. In this example, the restriction endonuclease *Mly*I is critical to triplet nucleotide substitution but other restriction endonucleases with properties similar to that of *Mly*I can be used providing the appropriate steps are modified and extra steps added if required, as will be understood by the skilled person.

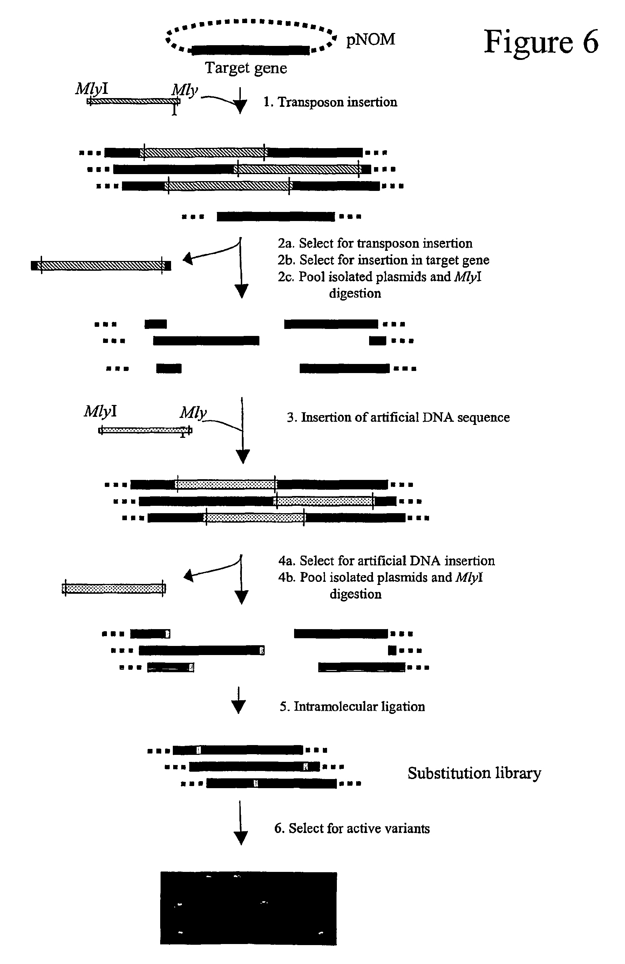

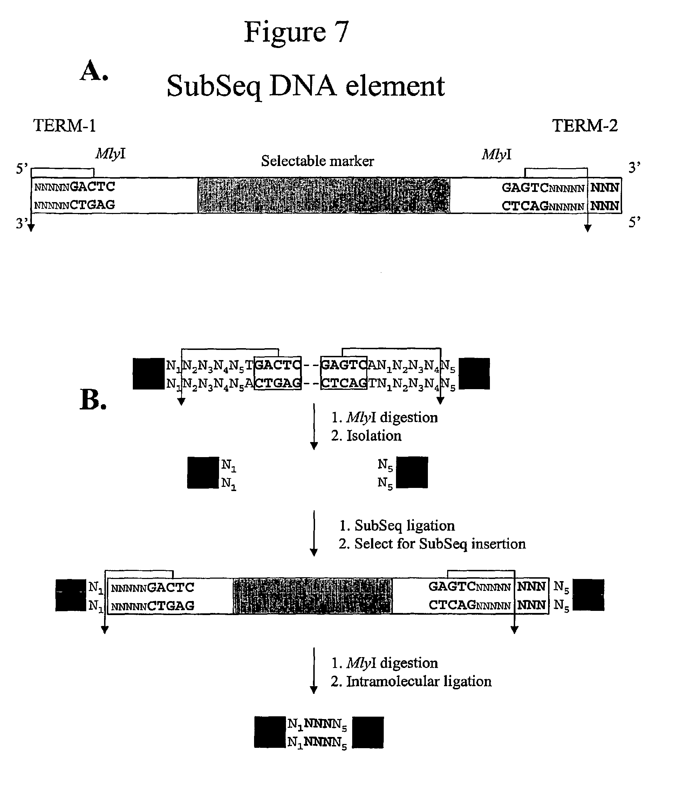

The example follows the procedure that is outlined in Figure 6 and is similar to the procedure outlined in Example 1. Both pNOM and MuDel are as outlined in Example 1. The MuDel was used in the same manner as in Example 1 and the same approach was taken: insertion of the transposon into the target gene; selection for the transposon-inserted target gene; removal of the transposon by restriction digestion. The next stage differs from Example 1 in that the intramolecular ligation was replaced by an intermolecular ligation, as outlined in Figure 6. At Step 3, intramolecular ligation is replaced by the intermolecular ligation of an artificial DNA sequence (e.g. SubSeq; see Figure 7). The target DNA sequence containing the artificial DNA sequence is selected for using a selectable marker present in the artificial DNA sequence and the plasmid DNA isolated and digested with *Mly*I (Step 4 of Figure 6). Intramolecular ligation results in the reformation of the *bla* gene, with three nucleotide base pairs substituted (Step 5). The resulting library is subjected to a selection or screen to select those variants with the required properties.

In Figure 6, hatched blocks represent the transposon, black blocks the target gene, speckled blocks the artificial DNA sequence, grey blocks the substitution point and the thick dashed lines the rest of the plasmid backbone. A new DNA sequence was inserted using standard DNA ligation techniques to contain a DNA element with the properties as illustrated in Figure 7A. The two different termini of the DNA sequence are marked TERM-1 and TERM-2 in Figure 7A. The last three nucleotide base pairs of TERM-2 can be a defined triplet sequence, fully random (that is every position can have the four possible nucleotides) or semi-random (that is that some positions may have the nucleotide allowed restricted). The gene that encodes the selectable marker is located between TERM-1 and TERM-2. The mechanism used is outlined in Figure 7B. MuDel transposon insertion results in a five bp duplication after gap repair in *E. coli.* The cleavage site of *Mly*I is 1 bp away from the recognition sequence resulting in the removal of 4 bp of the target gene at both ends, deleting the equivalent of 3 nucleotide base pairs from the target gene. The SubSeq DNA is ligated into the target gene at the cleavage point and those target genes with SubSeq inserted within them are selected using the selectable marker after transformation. Digestion of the SubSeq inserted target gene with *Mly*I results in the removal of SubSeq DNA except for the last three nucleotide base pairs at TERM-2. Intramolecular ligation results the reformation of the target gene but with three nucleotide base pairs replaced. The three substituted nucleotide base pairs are shown in bold.

### Creation of SubSeq

The DNA element described in Figure 7 (from hereon known as SubSeq) contains two *Mly*I sites, but other restriction endonucleases with properties similar to that of *Mly*I can be used providing the appropriate steps are modified and extra steps added if required, as will be understood by the skilled person. One *Mly*I site was placed 5 bp from one terminal of the DNA sequence (from hereon known as TERM-1) and the other *Mly*I site was placed 8 bp away from the other terminal (from hereon known as TERM-2). Linking the two *Mly*I sites is an appropriate selectable marker gene that provides a chosen strain of *E. coli* or any other suitable organism with a selection advantage under a particular condition, so making the organism viable or displaying a characteristic that will differentiate it from other cells that do not contain the SubSeq DNA element. The last three nucleotide base pairs of TERM-2 can be a defined triplet sequence, fully random (that is every position can have the four possible nucleotides) or semi-random (in that some positions may have the nucleotide allowed restricted).

Unless otherwise stated, all PCR reactions were performed with the Extensor Hi-Fidelity PCR Enzyme mix and performed as described above. The SubSeq DNA element was constructed by PCR using the oligonucleotide primers DDJdi017 (5' Phos-CGACCGAcTcAATACCTGTGACGGAAGATC 3' (SEQ ID NO:47); "Phos" signifies a phosphorylated nucleotide) and DDJdi018 (5' Phos-NNNAACTGGaC TCAGGCATTTGAGAAGCACAC 3' (SEQ ID NO:48); "Phos" signifies a phosphorylated nucleotide and "N" signifies any oligonucleotide) as the forward and reverse primers with 0.1 ng of the original mini-Mu (*Cam*^{R}-3) transposon acting as template, to create SubSeq. The 1095 bp PCR product was purified.

### Creation and sequencing of amino acid substitution library

An expanded library based on that created in Example 1 was used in this example. This expanded library contained up to 176 clones with MuDel inserted within the *bla* gene. The colonies containing MuDel within the *bla* gene of pNOM were pooled and plasmid DNA isolated as outlined in Example 1. The purified plasmid library was cut with *Mly*I and linear pNOM plasmid minus MuDel was isolated and purified after agarose gel electrophoresis as outlined in the Example 1. Prior to agarose gel electrophoresis, the plasmid DNA was dephosphorylated using calf intestinal alkaline phosphatase (NE BioLabs).

The SubSeq DNA sequence (50 ng) created as outlined above was ligated into the *Mly*I-digested pNOM (approx. 30 ng) using T4 DNA ligase. Up to 2 µl of the ligation mix was used to transform *E*. *coli* DH5α cells by electroporation and the cells plated on 20 µg/ml chloramphenicol LB agar plates to select for cells containing SubSeq inserted within the *bla* gene of pNOM. 192 colonies were selected at random from the chloramphenicol LB agar plates and grown in 96 deep-well culture plates containing 20 µg/ml chloramphenicol LB broth. Equal volumes were taken out of each well and pooled together.

The SubSeq-containing pNOM library was purified from cells as outlined in Example 1. Approximately 2 µg of the library was subjected to *Mly*I digestion as outlined above. That digestion removed SubSeq, resulting in the replacement of the 3 bp of the wild-type *bla* gene that were deleted on removal of MuDel earlier in the procedure. The band corresponding to linear pNOM (2115 bp) was isolated and purified after agarose gel electrophoresis.

The library of linear pNOM plasmids (10 ng) was subjected to intramolecular ligation using T4 DNA ligase (as described above) to rejoin the ends of the plasmid and constituted the BLA^{SUB} library. One tenth of the ligation mixture was used to transform DH5α and the cells plated on 15 µg/ml ampicillin LB agar plates to select for active TEM-1 β-actamase variants. More than 1000 colonies grew on the plate.

PCR using *Taq* DNA polymerase was performed on several randomly chosen colonies capable of growth on 15 µg/ml ampicillin using primers DDJdi010 and DDJdi011, as outlined in Example 1. The size of each of the products was 1070 bp, as expected. The DNA produced by the PCR were purified and sequenced using the oligonucleotide DDJdi010 as the primer. The exact nature of the mutations are shown in Table 4. This data shows that the amino acid substitutions can be incorporated at random positions in a protein using this transposon-based technology.

**Table 4. Sequence analysis of the bla gene with 3 bp substitution at random positions. The nucleotide base pairs exchanged are shown in bold. The change with relation to the amino acid sequence is also shown, with the amino acid sequences numbered using the recommended numbering systems (Ambler, R.P. et al. (1991) Biochem J. 276 (Pt 1) 269-270).**

| **Wild-type sequence** | **Substitution sequence** | **Amino acid substitutions** |
|---|---|---|
| CACAACATGGGG | CACAACACGCGG | M155T-G156R |
| CAGATCGCTGAG | CAGATCGCGTCG | E281S |
| ACGATGCCTGTA | ACGATGCCATCA | V184S |
| GCTTCCCGGCAA | GCTTCCGCTCAA | R204A |
| ATGCCTGTAGCA | ATGCCCAGAGCA | V184A |
| GCCATAACCATG | GCCATAAACTTG | T128N-M129L |
| GACTGGATGGAG | GACTGGACTAAG | M211T-E212K |
| GCTGAAGATCAG | GCTGAAGAGTAG | D38E-Q39stop |
| GAGCAACTCGGT | GAGCAACAACGT | L91Q-G92R |

### Example 4

This example is an expansion on example 3 and incorporates additional features into the SubSeq DNA sequence. Example 4 follows the same steps as outlined in Example 3 except for the differences described. The main difference is the nature of the SubSeq DNA element.

In this alternative to Example 3, the *Mly*I sites at TERM-1 and/or TERM-2 were shifted within the SubSeq sequence. Shifting the *Mly*I sequences to the appropriate positions can result in:
(i) Further deletion of another triplet or multiple triplet nucleotides;
(ii) Substitution of a triplet (3) nucleotide sequence with a quadruplet (4) nucleotide sequence;
(iii) Further insertion of another triplet or multiple triplet nucleotides.

### Example 5

This example illustrates how to create a library of variants containing insertions of amino acid sequences (e.g. whole proteins, protein domains or fragments (such as epitopes) of protein domains) at a random position in the target protein of interest using the transposon-based technology as outlined in Example 1. This example uses the MuDel transposon (Example 1) and the newly constructed pNOM plasmid, or a suitable derivative of the pNOM plasmid. Other transposons described in this specification can also be used in the procedure, with suitable modifications to the procedure, as will be understood by the skilled person. In this example, the restriction endonuclease *Mly*I is critical to domain insertion but other restriction endonucleases with properties similar to that of *Mly*I can be used providing the appropriate steps are modified and extra steps added if required, as will be understood by the skilled person.

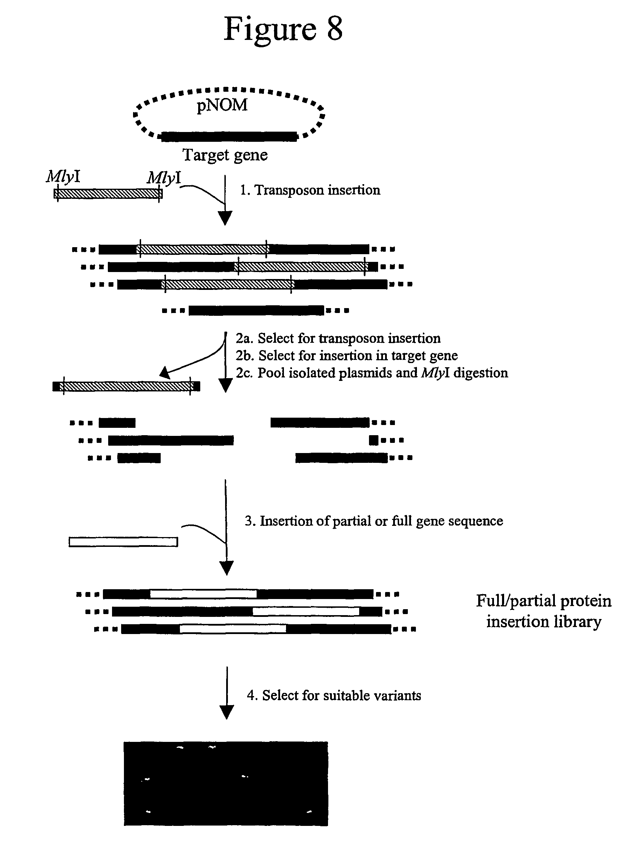

The example follows the procedure outlined in Figure 8 and is similar to the procedure outlined in Example 1. As before, the procedure comprises 4 main steps:
Step 1: The MuDel transposon (hatched blocks in Figure 8) is inserted into the target plasmid or target gene.
Step 2: Cells containing a plasmid-integrated MuDel are selected using the properties of the selectable marker gene. The plasmids are isolated and pooled, and the transposon is removed by *Mly*I digestion.
Step 3: The DNA sequence (clear blocks in Figure 8) is inserted into the target gene. In this case, the DNA to be inserted is the gene *cybC* which encodes the protein cytochrome *b*₅₆₂ (from hereon known as cyt b).
Step 4: The library is subjected to a selection or screening step that is suitable to identify proteins encoded by the chimeric gene with the desired properties.

Both pNOM and MuDel are identical to that as outlined in previous examples. MuDel was used in the same manner as in Examples 1 and 3 and the same approach was taken: insertion of the transposon into the target gene; selection for the transposon-inserted target gene; removal of the transposon by restriction digestion.

The library of MuDel inserted within the *bla* gene of pNOM used in this example is identical to that used in Example 3. The production of linear, dephosphorylated pNOM minus MuDel is exactly same as outlined in Example 3.

### Construction of the Cyt b insert.

Three different versions of the *cybC* gene that encode cyt b were created. As outlined in Example 1, the transposon can insert at three different positions with respect to one codon. As the introduced single break after transposon removal can occur at three different positions with respect to a single codon, the use of only a single open reading frame (ORF) for the *cybC* gene insert would make ²/₃ of the library redundant due to frameshifts. Therefore, two further versions of *cybC* were used with additional bases added to both ends to allow the sampling of all three ORFs. These constituted three separate libraries. Furthermore, for TEM-1 to tolerate the insertion of cyt b, a short linker may be required at one or both connection points. Therefore, each ORF version of *cybC* was composed of four different sequences that encode cyt b with either no linker or a linker sequence encoded in the primer oligonucleotides listed below, at either one or both termini of the gene.

Unless otherwise stated, all PCR reactions were performed with the Extensor Hi-Fidelity PCR Enzyme mix and its supplied buffers, as outlined above. Each ORF library of the *cybC* gene (ORFI, ORFII and ORFIII) was constructed using PCR using the *cybC* gene as the template as follows:
ORFI: Forward primers DDJlacB005 (5' GCAGATCTTGAAGACAATATGGA 3'; SEQ ID NO:69), DDJdi023 (5' ggcggtagcGCAGATCTTGAAGACAATATGGA (SEQ ID NO:70); lowercase letters signify nucleotides encoding the linking regions) and reverse primers DDJlacB006 (5' CCTATACTTCTGGTGATAGGCGT; SEQ ID NO:71) and DDJdi024 (5' gctgccaccCCTATACT TCTGGTGATAGGCGT (SEQ ID NO:72); lowercase letters signify nucleotides encoding the linking regions).
ORFII: Forward primers DDJdi019 (5' CGCAGATCTTGAAGACAATATGGA 3' (SEQ ID NO:73); underlined nucleotides are extra nucleotides used to maintain the ORF) and DDJdi025 (5' CggcggtagcGCAGATCTTGAAGACAATATGGA 3' (SEQ ID NO:74); underlined nucleotides are extra nucleotides used to maintain the ORF and lowercase letters signify nucleotides encoding the linking regions), and reverse primers DDJdi020 (5' CTATACTTCTGGTGATAGGCGT 3'; SEQ ID NO:75) and DDJdi026 (5' ctgccaccCCTATACTTCTGGTGATAGGCGT 3' (SEQ ID NO:76); lowercase letters signify nucleotides encoding the linking regions).
ORFIII: Forward primers DDJdi021 (5' CTGCAGATCTTGAAGACAATATGGA 3' (SEQ ID NO:77); underlined nucleotides are extra nucleotides used to maintain the ORF) and DDJdi027 (5' CTggcggtagcGCAGATCTTGAAGACAATATGGA 3' (SEQ ID NO:78); underlined nucleotides are extra nucleotides used to maintain the ORF and lowercase letters signify nucleotides encoding the linking regions) and reverse primers DDJdi022 (5' TATACTTCTGGTGATAGGCGT 3'; SEQ ID NO:79) and DDJdi028 (5' tgccaccCCTATACTTCTGGTGATAGGCGT 3' (SEQ ID NO:80); lowercase letters signify nucleotides encoding the linking regions).

The 318-336 bp products were purified and a 5' phosphate group added to the PCR product using 20 units of T4 polynucleotide kinase in the T4 DNA ligase (NE Biolabs) reaction buffer.

### Creation and sequencing of cybC insertion libraries.

The next stage differs from Example 1 and more closely follows Example 3, in that the intramolecular ligation is replaced by an intermolecular ligation, as outlined in Figure 8. Instead of SubSeq being inserted at random positions of the *bla* gene, as in Example 3, the ORF libraries of *cybC* are inserted. Although *cybC* is inserted in this case, any DNA element that encodes either a whole gene, gene segment equivalent to a protein domain, gene segment equivalent to partial amino acid sequence of a whole protein or domain of a protein (for example an epitope), or any other amino acid sequence could be used.

An expanded library, based on that created in Example 1, that was used in Example 3 was also used in this example. This expanded library contained up to 176 clones with MuDel inserted within the *bla* gene. The colonies containing MuDel within the *bla* gene of pNOM were pooled and plasmid DNA isolated as outlined in Example 1. The purified plasmid library was cut with *Mly*I and linear pNOM plasmid minus MuDel was isolated and purified after agarose gel electrophoresis as outlined in the Example 1. Prior to agarose gel electrophoresis, the plasmid DNA was dephosphorylated using calf intestinal alkaline phosphatase.

The three ORF libraries of *cybC* (50 ng) created above were ligated separately into the *Mly*I-digested pNOM (*circa* 30 ng) using T4 DNA ligase. Up to 2 µl of the ligation mix of each reaction was used to transform *E. coli* DH5α cells by electroporation and the cells plated on 15 µg/ml ampicillin LB agar plates to select for cells containing active chimeric cyt b-TEM-1 proteins. Only 8 colonies grew on the control plate (cells transformed with a ligation containing no ORF library insert), whereas 45, 130 and 150 colonies grew on the plate representing ORFI, ORFII and ORFIII *cybC* libraries, respectively.

PCR using *Taq* DNA polymerase was performed on 10, 15 and 10 randomly chosen colonies from the plates representing ORFI, ORFII and ORFIII, respectively, using primers DDJdi010 and DDJdi011, as outlined in Example 1. The size of the products ranged from 1300 bp to 1600 bp. The DNA produced by the PCR were purified and sequenced using the oligonucleotide DDJdi1010 as the primer. The exact nature of the mutations are shown in Table 5. Some of the chimeras contained two *cybC* genes inserted in tandem at the same position in the *bla* gene. This data shows that the domain insertions can be incorporated at random positions in a protein using this transposon based-technology.

**Table 5. Sequence analysis of the bla-cybC gene chimeras. The ORF columns refers to the ORF library from which the genes where isolated. The ↓ refers to the point of insertion in either the bla gene or TEM-1 proteins. The N- and C-terminal linker columns refer to the amino acid sequence that links TEM-1 with cyt b. Those ORFs marked with an * indicate genes with a tandem insertion of cybC within bla. Several of the C-terminal linker sequences could not be determined due to poor sequence data at these regions as a result of low signal because of the distance away from the priming site.**

| **ORF** | **Insertion point in *bla*** | **Insertion point in TEM-1** | **N-terminal linker** | **C-terminal linker** |
|---|---|---|---|---|
| I | 622-TGGATGGAG↓ | E212↓ | GGS | GGS |
| I | 64-TTTGCTCAC↓ | H26↓ | GGS | GGS |
| I | 505-GAAGCCATA↓ | I173↓ | GS | None |
| II | 322-GAAAAGCAT-CT↓ | L113↓ | GGS | GGR |
| II | 568-AAACTATTA-AC↓ | L194↓ | T | R to S cyt |
| II | 328-CATCTTACG-GA↓ | T114↓ | D | R to S cyt |
| II | 781-ACGACGGGC-AG↓ | G267↓ | S-GGS | Unknown |
| II | 493-CCGGAGCTG-AA↓ | L169↓ | N-GGS | Unknown |
| II* | 328-CATCTTACG-GA↓ | T114↓ | D | Unknown |
| III | 325-AAGCATCTT-A↓ | L113↓ | T | GGN |
| III* | 325-AAGCATCTT-A↓ | L113↓ | T | Unknown |

The skilled person will understand that the method outlined in this Example can be used as a tool to create domain insertion so as to generate a molecular switch, as outlined in the "Background" section above.

### Example 6

This example describes alternatives to the MuIns transposon in the previous examples. In every previous example containing MuIns, the new transposon sequences described below replace MuIns in the scheme, together with the suitable changes in the procedure that will allow transposition to occur.

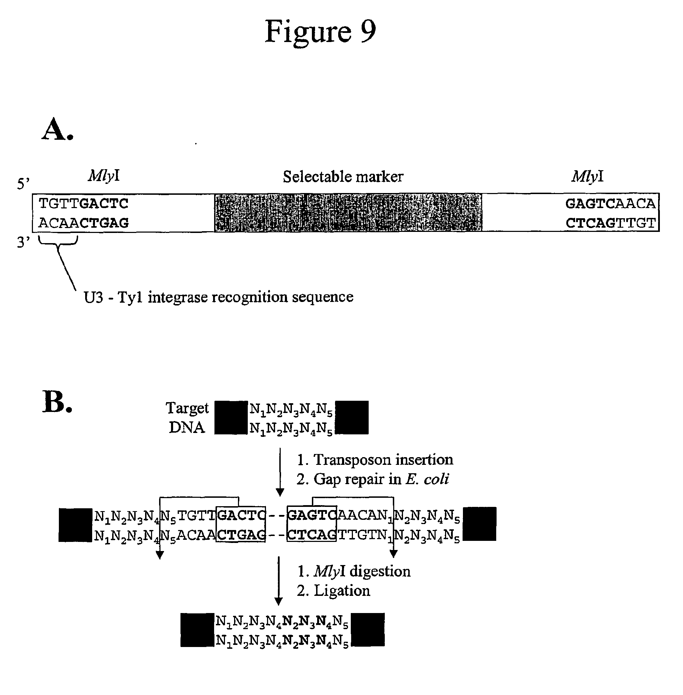

In the first instance, the AT-2 transposon, described by Devine & Boeke (Nucleic Acid Res. (1994) **22** 3765-3772) was modified, as shown in Figure 9A. The AT-2 transposon shows similar characteristics to mini-Mu and efficient transposition can be performed *in vitro.* The main difference is that the transposase recognition site consists of only the terminal four nucleotide base pairs. Placing the *Mly*I recognition site directly after this sequence allows insertion mutagenesis to proceed by the mechanism outlined in Figure 9B, without disruption to the transposition efficiency (inserted nucleotides are shown in bold). A selectable marker is present within the transposon between the two termini as illustrated in Figure 9A. The U3 sequences identified by the Ty1 integrase are indicated. A gene encoding a selectable marker will reside between the two terminal U3 and *Mly*I recognition sequences. The selectable marker is a gene that provides a chosen strain of *E. coli,* or any other suitable organism, with a selection advantage under a particular condition, so making the organism viable or displaying a characteristic that will differentiate it from other cells that do not contain the transposon sequence.

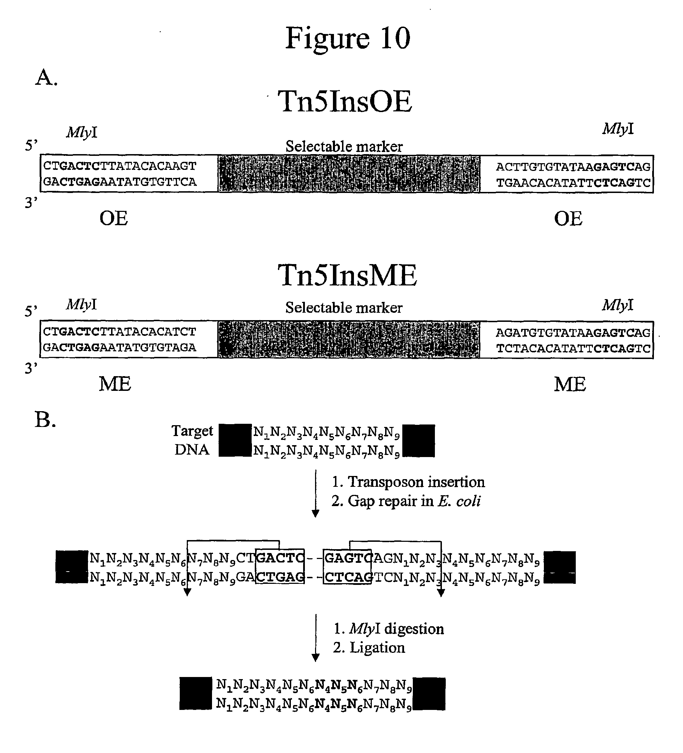

In the second instance, the Tn5 transposon, described by Goryshin & Reznikoff (J. Biol. Chem. (1998) 273 7367-7374), was adapted to replace the MuIns transposon, as shown in Figure 10A. The Tn5InsOE contains the OE (outside end) element and the Tn5InsME transposon contains the ME (mosaic end) element. These elements can promote transposition of DNA sequences that lie between them. In each case, a selectable marker gene lies between the two OE or ME elements. The selectable marker is a gene that encodes a protein that provides a chosen strain of *E. coli,* or any other suitable organism, with a selection advantage under a particular condition, so making the organism viable or displaying a characteristic that will differentiate it from other cells that do not contain the transposon sequence. Any modified version of the OE or ME elements that contains changes in its nucleotide sequence can be utilised, providing the sequence still contains the *Mly*I sequence at the required position and the DNA can still act as a transposon.

The mechanism by which triplet nucleotide insertion occurs when using Tn5InsOE or Tn5InsME is shown in Figure 10B. The inserted nucleotides are shown in bold. Unlike Mu and AT-2, Tn5 transposition occurs via a 9 nucleotide staggered cut. The *Mly*I recognition sequence is placed two nucleotide base pairs from each terminus and allows the precise insertion of three nucleotide base pairs upon *Mly*I digestion followed by intramolecular ligation.

### Example 7

This example illustrates how to create a library of variants containing triplet nucleotide additions at random positions in the *bla* gene using a transposon based technology utilising a modified transposon that contains modified recognition sites based on the Tn5 transposon, as shown in Figure 10. The chloramphenicol resistance gene is included as a selectable marker. This example uses two new transposons, termed Tn5InsOE and Tn5InsME and the pNOM plasmid but other suitable derivatives of pNOM can be used. In this example, the restriction endonuclease *Mly*I is critical to triplet nucleotide insertion but other restriction endonucleases with properties similar to that of *Mly*I can be used, providing the appropriate steps are modified and extra steps added if required, as will be understood by a skilled person.

This example follows the procedure outlined in Example 1 and Figure 2. The main difference is with respect to the transposon used. In summary, the procedure consists of 4 main steps:
Step 1: The Tn5InsOE or Tn5InsME transposon is inserted into the target plasmid or gene.
Step 2: Cells containing a plasmid-integrated Tn5InsOE or Tn5InsME contain the *Cam*^{R} gene and so can grow in the presence of chloramphenicol. The plasmids are isolated and pooled, and the transposon removed by *Mly*I digestion.
Step 3: Intramolecular ligation results in the reformation of the target gene, plus nucleotide base pairs.
Step 4: The resulting library is subjected to a selection or screen to select those variants with the required properties.

In Figure 2, the hatched blocks represent the transposon, solid blocks the *bla* gene, gaps and grey blocks the insertion point and the thick dashed lines the rest of the plasmid backbone.

The procedure can also be applied to a target gene other than the *bla* gene, provided that:
1. there are suitable modifications to the selection or screening step at step 4 in Figure 2 that are suitable to the protein encoded by the target gene; and
2. any undesirable restriction sites are either not present or removed from the target gene.

Describing this example now in detail, a modified mini-Mu transposon containing a Tn5-derived sequence is used, along with a newly constructed pNOM plasmid (see example 1). The resultant transposon DNA is derived from mini-Mu apart from those sequences required for the transposition reaction (e.g. OE or ME sequences recognised by transposase enzymes) which is derived from Tn5. In this example, the restriction endonuclease *Mly*I is critical to triplet nucleotide insertion but other restriction endonucleases with properties similar to that of *Mly*I can be used, providing the appropriate steps are modified and extra steps added if required, as will be understood by the skilled person. Similarly, the DNA sequence between the OE or ME sequences can be altered, provided that it comprises a sequence for an appropriate selectable marker.

### Construction of Tn5InsOE and Tn5InsME

The original Mu phage-derived transposon, mini-Mu (*Cam*^{R})*,* was engineered for use in the creation of the random triplet nucleotide addition. In this case, the *Cam*^{R} gene is used as a selectable marker within the transposon. This can be exchanged for another gene that will provide a chosen strain of *E. coli* or any other suitable organism with a selection advantage under a particular condition so making the organism viable or displaying a characteristic that will differentiate it from other cells that do not contain the transposon sequence.

The ability to duplicate nucleotide triplets depends on the transposon insertion mechanism and the position of two introduced restriction sites, as outlined in Figure 10. The mini-Mu transposon was engineered so as to act as a vehicle for the insertion of specific restriction sites into the target gene (Figure 3A). The restriction endonuclease chosen was *Mly*I*,* a type IIS enzyme that cuts 5 bp outside its recognition sequence to generate a blunt end (cleavage profile 5' GAGTC(N₅)↓ 3'). The nucleotide sequences towards the two termini of the mini-Mu transposon were replaced by a sequence based on the Outside End (OE) or Mosaic End (ME) sequence from the Tn5 transposon. This new nucleotide sequence now requires the Tn5 transposase for insertion into the target DNA. Transposition of Tn5Ins will occur via a 9 bp staggered cut in the target DNA that, following *E. coli* gap repair, results in the duplication of these 9 bp (Figure 10B). Digestion of the DNA with *Mly*I removes the transposon along with three additional nucleotide base pairs from the target gene at both termini. Intramolecular ligation of the two blunt ends results in the in-frame duplication of 3 nucleotides from the target gene (Figure 10B).

Unless otherwise stated, all PCR reactions were performed with the Extensor Hi-Fidelity PCR Enzyme mix and performed as described above. The Tn5Ins transposon was constructed by PCR using the oligonucleotide primer AS1 (5' CTGACTCTTATACACAAGTCGCGAAAGCGTTTCACGATA 3'; SEQ ID NO:89) or AS2 (5' CTGAGTCTTATACACATCTCGCGAAAGCGTTTCACGATA3'; SEQ ID NO:90) as both forward and reverse primer with 0.1 ng of the original mini-Mu (*Cam*^{R}-3) transposon acting as template, to create transposons Tn5InsOE and Tn5InsME, respectively. The 1302 bp product was purified ready for use in a transposition reaction.

### Transposition reaction and transformation into E. coli cells.

Transposition with Tn5InsOE and Tn5InsME was performed at 37°C for 2 hr followed by heat inactivation for 10 min at 70°C. The reaction mixture was composed of 1 µl 10x reaction buffer (500 mM Tris-acetate (pH 7.5), 1.5 M potassium acetate, 100 mM magnesium acetate, 40 mM spermidine), 200 ng pNOM, 0.232 pmoles Tn5Ins and 1 unit of EZ-Tn5™ transposase (Epicentre, Madison USA) in a total volume 10 µl. The reaction was stopped by the addition of 1 µl stop solution (1% SDS) prior to incubation at 70°C for 10 min.

Either 1 µl or 2 µl of the reaction mixture was used to transform *E. coli* DH5α cells by electroporation and the cells plated on LB agar containing 20 µg/ml chloramphenicol to select for cells containing the *Cam*^{R} gene and hence the Tn5Ins gene.

As mentioned above, the general outline of the method for the creation of triplet nucleotide insertions at random positions within a target gene is shown in Figure 2. The *bla* gene that encodes TEM-1β-lactamase was chosen as the target, as before. The new vector, pNOM, was used as the source of the *bla* gene and therefore acts as the target DNA for Tn5Ins insertion

As an alternative to the above description, a gene of interest independent of pNOM can be used as the target for transposon insertion. If required, the gene of interest can be cloned into pNOM or another suitable vector using standard techniques after transposon insertion. Alternatively, after transposon insertion into the gene of interest, the gaps present in the DNA strands formed as a result of the transposition reaction that are normally repaired in the organism can be repaired *in vitro* using the appropriate gap repair and ligation techniques.

The place of insertion of Tn5InsOE or Tn5InsME into pNOM should be distributed evenly throughout the plasmid and so a strategy is required that will select for cells containing the transposon inserted in to the *bla* gene region. The transposition of the transposon into the plasmid DNA confers resistance to chloramphenicol on *E. coli,* allowing for selection of cells containing Tn5InsOE- or TnSInsME-inserted pNOM. Those colonies that have Tn5InsOE or Tn5InsME inserted within the *bla* gene region will disrupt TEM-1 expression and thus affect the cells' ability to grow in the presence of ampicillin.

### Selection of colonies with transposon-disrupted bla gene

After transformation of *E. coli* DH5a with 1 µl of the transposition reaction and plating ¹/₂ of the transformation mix on 20 µg/ml chloramphenicol, over 200 colonies were observed when Tn5InsME was used and 20 colonies when Tn5InsOE used. From this point onwards, the library created with Tn5InsME was utilised. To select for Tn5InsME transposons inserted with the *bla* gene, 96 colonies were selected that grew on 20 µg/ml chloramphenicol and replated on both a 100 µg/ml ampicillin and a 20 µg/ml chloramphenicol LB agar plates. Of the 96 colonies, 66 grew only on the chloramphenicol plate and were deemed to have a disrupted *bla* gene due to transposon insertion in this region.

The pNOM plasmid with TN5InsME inserted within the *bla* gene was purified individually from 10 of the 66 colonies. Each of the plasmids was subjected to digestion with *Mly*I, followed by agarose gel electrophoresis. The band corresponding to the linear pNOM minus Tn5InsME was isolated and purified after agarose gel electrophoresis. Intramolecular ligation was performed using T4 DNA ligase and approximately 10 ng of linear DNA. Up to 2 µl of the ligation mix was used to transform *E. coli* DH5α cells by electroporation and the cells plated on 15 µg/ml ampicillin LB agar plates to select for cells containing an active TEM-1 β-lactamase. The *bla* gene in each of the individual plasmids were also sequenced to determine the nature of insertion, using the primer DDJdi010. These sequences are shown in Table 6 below. The amino acid duplications were found to be present throughout TEM-1. Only one sequence was present twice in more than clone. This successfully demonstrates the use of the transposon-based method to incorporate amino acid insertions into a target gene of interest.

**Table 6. Sequence analysis of the TEM-1 amino acid insertion library. The residue that is inserted is underlined in the mutation column. The mutation labelled with * was found in two different clones. The ability of the TEM-1 insertion variant to confer resistance to 15 µg/ml ampicillin on E. coli was used as the criteria as to whether the variant was active or not. The Amp MIC refers the ampicillin minimum inhibitory concentration that prevents cell growth.**

| **Wild-type** | **Mutation** | **TEM-1 activity?** | **Amp MIC (µg/ml)** |
|---|---|---|---|
| 45-GYI | 45-GYYI | No | - |
| 77-CGA | 77-CGGA | No | - |
| 78-GAV | 78-GAAV | Yes | 8000 |
| 80-VLS | 80-VLLS* | No | - |
| 121-ELC | 121-ELLC | Yes | 8000 |
| 243-SRG | 243-SPRG | No | - |
| 249-ALG | 249-ALLG | No | - |
| 250-LGP | 250-LGGP | No | - |
| 257-PSR | 257-PSSR | No | - |

## Claims

1. Method for altering the amino acid sequence of a target polypeptide by altering a target DNA sequence which encodes that polypeptide, the method comprising the step of introducing a transposon into the target DNA sequence, in which the transposon comprises a first restriction enzyme recognition sequence towards each of its termini, the recognition sequence not being present in the remainder of the transposon, or in the target DNA sequence, or in a construct comprising the target DNA sequence, the first restriction enzyme recognition sequence being recognised by a first restriction enzyme which is an outside cutter and being positioned such that the first restriction enzyme has a DNA cleavage site positioned beyond the end of the terminus of the transposon.

2. Method according to claim 1 wherein the amino acid sequence is altered by the deletion, insertion or substitution of at least one amino acid.

3. Method according to any preceding claim wherein at least one amino acid is inserted into the amino acid sequence of the target polypeptide.

4. Method according to claim 3 wherein a single amino acid is inserted into the amino acid sequence of the target polypeptide.

5. Method according to claim 1 or 2 wherein at least one amino acid is deleted from the amino acid sequence of the target polypeptide.

6. Method according to claim 5 wherein a single amino acid is deleted from the amino acid sequence of the target polypeptide.

7. Method according to any of claims 3 to 6 comprising the following steps:
a) conducting a transposition reaction comprising mixing the transposon, the target DNA and a transposase enzyme;
b) digestion of DNA resulting from (a) with a first restriction enzyme which recognises the first restriction enzyme recognition sequence contained in the transposon;
c) separation of DNA which does not comprise the transposon;
d) conducting an intramolecular ligation reaction of the DNA from (c); and
e) expression of protein from the DNA from (d).

8. Method according to claim 1 or 2 wherein at least one amino acid of the amino acid sequence of the target polypeptide is substituted with a different amino acid.

9. Method according to claim 8 wherein a single amino acid of the amino acid sequence of the target polypeptide is substituted with a different amino acid.

10. Method according to claim 8 or 9 comprising the following steps:
a) conducting a transposition reaction comprising mixing the transposon, the target DNA and a transposase enzyme;
b) digestion of DNA resulting from (a) with a first restriction enzyme which recognises the first restriction enzyme recognition sequence contained in the transposon;
c) separation of DNA which does not comprise the transposon;
d) conducting an intermolecular ligation of DNA from (c) with a second DNA sequence comprising at least two second restriction enzyme recognition sites located such that at least one of the cleavage sites is not at a terminus of the second DNA sequence;
e) conducting the transformation of a host organism with DNA from (d) and selecting cells containing the second DNA sequence;
f) isolating DNA from cells selected in (e) and digestion of that DNA with a second restriction enzyme which recognises the second restriction enzyme recognition sites, the second restriction enzyme being an outside cutter;
g) conducting an intramolecular ligation of DNA from (f); and
h) expression of protein from the DNA from (g).

11. Method according to claim 10 wherein the second restriction enzyme is the same as the first restriction enzyme.

12. Method according to claim 10 or 11 wherein the second DNA sequence comprises a gene which gives a host cell containing the second DNA sequence a selectable characteristic compared to a cell not containing the second DNA sequence.

13. Method according to claim 1 or 2 wherein the amino acid sequence of the target polypeptide is altered by the insertion of a further amino acid sequence.

14. Method according to claim 13 comprising the following steps:
a) conducting a transposition reaction comprising mixing the transposon, the target DNA and a transposase enzyme;
b) digestion of DNA resulting from (a) with a first restriction enzyme which recognises the first restriction enzyme recognition sequence contained in the transposon;
c) separation of DNA which does not comprise the transposon;
d) conducting an intermolecular ligation of DNA from (c) with a third DNA sequence encoding for a further amino acid sequence; and
e) expression of protein from the DNA from (d).

15. Method according to claim 13 or 14 wherein the further amino acid sequence is a full protein, a protein domain or a protein fragment.

16. Method according to claim 15 wherein the protein fragment is an epitope.

17. Method according to claim 15 wherein the protein fragment is a binding domain.

18. Method according to claim 15 wherein the protein fragment is an allosteric site.

19. Method according to claim 15 wherein the protein fragment is a defined functional region.

20. Method according to claim 15 wherein the protein fragment is an oligomerisation interface.

21. Method according to any of claims 14-20 wherein the third DNA sequence comprises a gene which gives a host cell containing the third DNA sequence a selectable characteristic compared to a cell not containing the third DNA sequence.

22. Method according to any of claims 14-21 wherein the third DNA sequence has an open reading frame which is the same as that of the target DNA.

23. Method according to any of claims 14-22 wherein the third DNA sequence contains a stop codon.

24. Method according to any of claims 14-23 wherein the third DNA sequence contains an initiation codon.

25. Method according to any preceding claim wherein the first restriction enzyme is a Type IIS enzyme.

26. Method according to claim 25 wherein the first restriction enzyme is *Mly*I.

27. Method according to any preceding claim wherein the transposon has a low target site preference.

28. Method according to claim 27 wherein the transposon is derived from one of: mini-Mu, AT-2 or Tn5.

29. Method according to any preceding claim wherein the transposon comprises a gene which gives a host cell containing the transposon a selectable characteristic compared to a cell not containing the transposon.

30. Method according to claim 27 wherein the transposon comprises the DNA sequence 5'-NGACTC-3' as the 5' terminal and 5'-GAGTCN-3' as the 3' terminal, or comprises the DNA sequence 5'-NNNNGACTC-3' as the 5' terminal and 5'-GAGTCNNNN-3' as the 3' terminal, or comprises the DNA sequence 5'-TGTTGACTC-3' as the 5' terminal and 5'-GAGTCAACA-3' as the 3' terminal, or comprises the DNA sequence 5'-CTGACTC-3' as the 5' terminal and 5'-GAGTCAG-3' as the 3' terminal.

31. Method according to any preceding claim wherein the target DNA is carried in a plasmid.

32. Method according to claim 31 wherein the plasmid is pNOM or a derivative thereof.

33. Transposon comprising a restriction enzyme recognition sequence towards each of its termini, the recognition sequence not being present in the remainder of the transposon, being a recognition sequence for a restriction enzyme which is an outside cutter and being positioned such that the restriction enzyme has a DNA cleavage site positioned beyond the end of the terminus of the transposon.

34. Transposon according to claim 33 suitable for use in a method according to any of claims 1-32.

35. Transposon according to claims 33 or 34 wherein each restriction enzyme recognition sequence is located between 1 and 20 nucleotides from a transposon terminus.

36. Transposon according to claim 35 wherein each restriction enzyme recognition sequence is located at 1, 2, 3, 4 or 5 nucleotides from a transposon terminus

37. Transposon according to any of claims 33 to 36 wherein the restriction enzyme is *Mly*I.

38. Transposon according to claim 37 comprising the DNA sequence 5'-NGACTC-3' as the 5' terminal and 5'-GAGTCN-3' as the 3' terminal.

39. Transposon according to claim 37 comprising the DNA sequence 5'-NNNNGACTC-3' as the 5' terminal and 5'-GAGTCNNNN-3' as the 3' terminal.

40. Transposon according to claim 37 comprising the DNA sequence 5'-TGTTGACTC-3' as the 5' terminal and 5'-GAGTCAACA-3' as the 3' terminal.

41. Transposon according to claim 37 comprising the DNA sequence 5'-CTGACTC-3' as the 5' terminal and 5'-GAGTCAG-3' as the 3' terminal.

42. Transposon according to claim 40 or 41 comprising at least one variation in the 5' terminal and/or 3' terminal DNA sequence, wherein the transposon is viable for transposition.

43. Plasmid having the DNA sequence shown in Figure 1.

44. Plasmid having a DNA sequence which has been adapted from the sequence shown in Figure 1 by:
a) silent mutations in the DNA sequence,
b) substitution of the *bla* gene with an alternative selectable marker,
c) by alteration of non-essential elements of the DNA sequence, or
d) by an additional DNA sequence of interest inserted at a point in the DNA sequence of Figure 1.

45. Kit comprising a transposon according to any of claims 33-42.

46. Kit according to claim 45 further comprising a plasmid according to claim 43 or 44.

47. Kit according to claim 45 or 46 further comprising a transposase.

48. Kit according to any of claims 45-47 further comprising at least one buffer.

49. Kit according to any of claims 45-48 further comprising at least one oligonucleotide.

50. Kit according to any of claims 45-49 for use in the method according to any of claims 1 to 32.

51. Method of determining whether the introduction of a mutation into a target polypeptide alters a detectable activity of that polypeptide, comprising the method of any of claims 1 to 32 and the further steps of:
a) screening for a difference in the activity of the altered target polypeptide compared to the unaltered target polypeptide; and
b) sequencing the altered target polypeptide to determine the location of the amino acid insertion, deletion or substitution.

## Patentansprüche

1. Verfahren zum Verändern der Aminosäuresequenz eines Targetpolypeptids durch Verändern einer Target-DNA-Sequenz, die das Polypeptid codiert, wobei das Verfahren den Schritt umfasst: Einbauen eines Transposons in die Target-DNA-Sequenz, wobei das Transposon an jedem seiner Termini eine Erkennungssequenz für ein erstes Restriktionsenzym umfasst, wobei die Erkennungssequenz im restlichen Transposon oder in der Target-DNA-Sequenz oder in einem Konstrukt, das die Target-DNA-Sequenz umfasst, nicht vorkommt, wobei die Erkennungssequenz für das erste Restriktionsenzym von einem ersten Restriktionsenzym erkannt wird, das außerhalb der Erkennungssequenz schneidet, und derart positioniert ist, dass das erste Restriktionsenzym eine DNA-Spaltstelle hat, die außerhalb des Endes des Terminus des Transposons liegt.

2. Verfahren nach Anspruch 1, wobei die Aminosäuresequenz durch die Deletion, Insertion oder Substitution wenigstens einer Aminosäure verändert wird.

3. Verfahren nach einem der vorhergehenden Ansprüche, wobei wenigstens eine Aminosäure in die Aminosäuresequenz des Targetpolypeptids inseriert wird.

4. Verfahren nach Anspruch 3, wobei eine einzelne Aminosäure in die Aminosäuresequenz des Targetpolypeptids inseriert wird.

5. Verfahren nach Anspruch 1 oder 2, wobei wenigstens eine Aminosäure aus der Aminosäuresequenz des Targetpolypeptids deletiert wird.

6. Verfahren nach Anspruch 5, wobei eine einzelne Aminosäure aus der Aminosäuresequenz des Targetpolypeptids deletiert wird.

7. Verfahren nach einem der Ansprüche 3 bis 6, umfassend die folgenden Schritte:
a) Durchführen einer Transpositionsreaktion, umfassend Mischen des Transposons, der Target-DNA und eines Transposaseenzyms;
b) Verdau von aus (a) resultierender DNA mit einem ersten Restriktionsenzym, das die in dem Transposon enthaltene Erkennungssequenz für das erste Restriktionsenzym erkennt;
c) Abtrennen von DNA, die das Transposon nicht umfasst;
d) Durchführen einer intramolekularen Ligationsreaktion der DNA aus (c); und
e) Proteinexpression von der DNA von (d).

8. Verfahren nach Anspruch 1 oder 2, wobei wenigstens eine Aminosäure der Aminosäuresequenz des Targetpolypeptids durch eine andere Aminosäure substituiert wird.

9. Verfahren nach Anspruch 8, wobei eine einzelne Aminosäure der Aminosäuresequenz des Targetpolypeptids durch eine andere Aminosäure substituiert wird.

10. Verfahren nach Anspruch 8 oder 9, umfassend die folgenden Schritte:
a) Durchführen einer Transpositionsreaktion, umfassend Mischen des Transposons, der Target-DNA und eines Transposaseenzyms;
b) Verdau von aus (a) resultierender DNA mit einem ersten Restriktionsenzym, das die in dem Transposon enthaltene Erkennungssequenz für das erste Restriktionsenzym erkennt;
c) Abtrennen von DNA, die das Transposon nicht umfasst;
d) Durchführen einer intermolekularen Ligation von DNA aus (c) mit einer zweiten DNA-Sequenz, die wenigstens zwei Erkennungsstellen für ein zweites Restriktionsenzym umfasst, die so lokalisiert sind, dass sich wenigstens eine der Spaltstellen nicht an einem Terminus der zweiten DNA-Sequenz befindet;
e) Durchführen der Transformation eines Wirtsorganismus mit DNA aus (d) und Selektieren von Zellen, die die zweite DNA-Sequenz enthalten;
f) Isolieren von DNA aus in (e) selektierten Zellen und Verdau dieser DNA mit einem zweiten Restriktionsenzym, das die Erkennungsstellen für das zweite Restriktionsenzym erkennt, wobei das zweite Restriktionsenzym außerhalb der Erkennungssequenz schneidet;
g) Durchführen einer intramolekularen Ligation von DNA aus (f); und
h) Proteinexpression von der DNA von (g).

11. Verfahren nach Anspruch 10, wobei das zweite Restriktionsenzym das gleiche ist wie das erste Restriktionsenzym.

12. Verfahren nach Anspruch 10 oder 11, wobei die zweite DNA-Sequenz ein Gen umfasst, das einer Wirtszelle, die die zweite DNA-Sequenz enthält, im Vergleich mit einer Zelle, die die zweite DNA-Sequenz nicht enthält, eine selektierbare Eigenschaft verleiht.

13. Verfahren nach Anspruch 1 oder 2, wobei die Aminosäuresequenz des Targetpolypeptids durch die Insertion einer weiteren Aminosäuresequenz verändert wird.

14. Verfahren nach Anspruch 13, umfassend die folgenden Schritte:
a) Durchführen einer Transpositionsreaktion, umfassend Mischen des Transposons, der Target-DNA und eines Transposaseenzyms;
b) Verdau von aus (a) resultierender DNA mit einem ersten Restriktionsenzym, das die in dem Transposon enthaltene Erkennungssequenz für das erste Restriktionsenzym erkennt;
c) Abtrennen von DNA, die das Transposon nicht umfasst;
d) Durchführen einer intermolekularen Ligation von DNA von (c) mit einer dritten DNA-Sequenz, die eine weitere Aminosäuresequenz codiert; und
e) Proteinexpression von der DNA von (d).

15. Verfahren nach Anspruch 13 oder 14, wobei die weitere Aminosäuresequenz ein vollständiges Protein, eine Proteindomäne oder ein Proteinfragment ist.

16. Verfahren nach Anspruch 15, wobei das Proteinfragment ein Epitop ist.

17. Verfahren nach Anspruch 15, wobei das Proteinfragment eine Bindungsdomäne ist.

18. Verfahren nach Anspruch 15, wobei das Proteinfragment eine allosterische Stelle ist.

19. Verfahren nach Anspruch 15, wobei das Proteinfragment eine definierte funktionelle Region ist.

20. Verfahren nach Anspruch 15, wobei das Proteinfragment eine Stelle zur Oligomerisation ist.

21. Verfahren nach einem der Ansprüche 14-20, wobei die dritte DNA-Sequenz ein Gen umfasst, das einer Wirtszelle, die die dritte DNA-Sequenz enthält, im Vergleich mit einer Zelle, die die dritte DNA-Sequenz nicht enthält, eine selektierbare Eigenschaft verleiht.

22. Verfahren nach einem der Ansprüche 14-21, wobei die dritte DNA-Sequenz einen offenen Leserahmen aufweist, der der gleiche ist, wie der der Target-DNA.

23. Verfahren nach einem der Ansprüche 14-22, wobei die dritte DNA-Sequenz ein Stoppcodon enthält.

24. Verfahren nach einem der Ansprüche 14-23, wobei die dritte DNA-Sequenz ein Startcodon enthält.

25. Verfahren nach einem der vorhergehenden Ansprüche, wobei das erste Restriktionsenzym ein Typ IIS-Enzym ist.

26. Verfahren nach Anspruch 25, wobei das erste Restriktionsenzym Mlyl ist.

27. Verfahren nach einem der vorhergehenden Ansprüche, wobei das Transposon eine geringe Präferenz für den Zielort hat.

28. Verfahren nach Anspruch 27, wobei das Transposon abstammt von einem von Mini-Mu, AT-2 oder Tn5.

29. Verfahren nach einem der vorhergehenden Ansprüche, wobei das Transposon ein Gen umfasst, das einer Wirtszelle, die das Transposon enthält, im Vergleich mit einer Zelle, die das Transposon nicht enthält, eine selektierbare Eigenschaft verleiht.

30. Verfahren nach Anspruch 27, wobei das Transposon die DNA-Sequenz 5'-NGACTC-3' als 5'-Terminus und 5'-GAGTCN-3' als 3'-Terminus umfasst, oder die DNA-Sequenz 5'-NNNNGACTC-3' als 5'-Terminus und 5'-GAGTCNNNN-3' als 3'-Terminus umfasst, oder die DNA-Sequenz 5'-TGTTGACTC-3' als 5'-Terminus und 5'-GAGTCAACA-3' als 3'-Terminus umfasst, oder die DNA-Sequenz 5'-CTGACTC-3' als 5'-Terminus und 5'-GAGTCAG-3' als 3'-Terminus umfasst.

31. Verfahren nach einem der vorhergehenden Ansprüche, wobei die Target-DNA von einem Plasmid getragen wird.

32. Verfahren nach Anspruch 31, wobei das Plasmid pNOM oder ein Derivat davon ist.

33. Transposon, das an jedem seiner Termini eine Erkennungssequenz für ein Restriktionsenzym umfasst, wobei die Erkennungssequenz im restlichen Transposon nicht vorkommt, eine Erkennungssequenz für ein Restriktionsenzym ist, das außerhalb der Erkennungssequenz schneidet, und derart positioniert ist, dass das Restriktionsenzym eine DNA-Spaltstelle hat, die außerhalb des Endes des Terminus des Transposons liegt.

34. Transposon nach Anspruch 33, das zur Verwendung in einem Verfahren nach einem der Ansprüche 1-32 geeignet ist.

35. Transposon nach den Ansprüchen 33 oder 34, wobei jede Erkennungssequenz für das Restriktionsenzym zwischen 1 und 20 Nukleotide entfernt von einem Transposonterminus lokalisiert ist.

36. Transposon nach Anspruch 35, wobei jede Erkennungsstelle für das Restriktionsenzym 1, 2, 3, 4 oder 5 Nukleotide entfernt von einem Transposonterminus lokalisiert ist.

37. Transposon nach einem der Ansprüche 33 bis 36, wobei das Restriktionsenzym Mlyl ist.

38. Transposon nach Anspruch 37, umfassend die DNA-Sequenz 5'-NGACTC-3' als 5'-Terminus und 5'-GAGTCN-3' als 3'-Terminus.

39. Transposon nach Anspruch 37, umfassend die DNA-Sequenz 5'-NNNNGACTC-3' als 5'-Terminus und 5'-GAGTCNNNN-3' als 3'-Terminus.

40. Transposon nach Anspruch 37, umfassend die DNA-Sequenz 5'-TGTTGACTC-3' als 5'-Terminus und 5'-GAGTCAACA-3' als 3'-Terminus.

41. Transposon nach Anspruch 37, umfassend die DNA-Sequenz 5'-CTGACTC-3' als 5'-Terminus und 5'-GAGTCAG-3' als 3'-Terminus.

42. Transposon nach Anspruch 40 oder 41, umfassend wenigstens eine Variation in der 5'-terminalen und/oder 3'-terminalen DNA-Sequenz, wobei das Transposon zur Transposition fähig ist.

43. Plasmid mit der in Figur 1 gezeigten DNA-Sequenz.

44. Plasmid mit einer DNA-Sequenz, die aus der in Figur 1 gezeigten Sequenz hergerichtet wurde durch:
a) stille Mutationen in der DNA-Sequenz,
b) Substitution des bla-Gens durch einen anderen selektierbaren Marker,
c) Verändern nicht-wesentlicher Elemente der DNA-Sequenz, oder
d) eine zusätzliche DNA-Sequenz von Interesse, die an einem Punkt in die DNA-Sequenz von Figur 1 inseriert ist.

45. Kit, umfassend ein Transposon nach einem der Ansprüche 33-42.

46. Kit nach Anspruch 45, weiterhin umfassend ein Plasmid nach Anspruch 43 oder 44.

47. Kit nach Anspruch 45 oder 46, weiterhin umfassend eine Transposase.

48. Kit nach einem der Ansprüche 45-47, weiterhin umfassend wenigstens einen Puffer.

49. Kit nach einem der Ansprüche 45-48, weiterhin umfassend wenigstens ein Oligonukleotid.

50. Kit nach einem der Ansprüche 45-49 zur Verwendung in dem Verfahren nach einem der Ansprüche 1 bis 32.

51. Verfahren zum Feststellen, ob der Einbau einer Mutation in ein Targetpolypeptid eine detektierbare Aktivität des Polypeptids verändert, umfassend das Verfahren nach einem der Ansprüche 1 bis 32 und die weiteren Schritte:
a) Screenen auf einen Unterschied in der Aktivität des veränderten Targetpolypeptids im Vergleich mit dem unveränderten Targetpolypeptid; und
b) Sequenzieren des veränderten Targetpolypeptids, um den Ort der Aminosäureinsertion, -deletion oder -substitution festzustellen.

## Revendications

1. Procédé visant à altérer la séquence d'acides aminés d'un polypeptide cible par le biais de l'altération d'un ADN cible codant pour ce polypeptide, le procédé comprenant l'étape consistant à introduire un transposon dans la séquence d'ADN cible, le transposon en question comprenant une première séquence de reconnaissance pour une enzyme de restriction au niveau de chacune de ses extrémités, la séquence de reconnaissance n'étant pas présente dans la partie restante du transposon ou dans la séquence de l'ADN cible ou dans une construction comprenant la séquence d'ADN cible, la première séquence de reconnaissance d'enzyme de restriction étant reconnue par une première enzyme de restriction, qui réalise une coupure externe, et étant situé de sorte que la première enzyme de restriction ait un site de clivage d'ADN placé au-delà de l'extrémité terminale du transposon.

2. Procédé selon la revendication 1, dans lequel la séquence d'acides aminés est altérée par le biais de la délétion, de l'insertion ou de la substitution d'au moins un acide aminé.

3. Procédé selon l'une quelconque des revendications précédentes, dans lequel au moins un acide aminé est inséré dans la séquence d'acides aminés du polypeptide cible.

4. Procédé selon la revendication 3, dans lequel un seul acide aminé est inséré dans la séquence d'acides aminés du polypeptide cible.

5. Procédé selon la revendication 1 ou 2, dans lequel au moins un acide aminé est délété d'une séquence d'acides aminés du polypeptide cible.

6. Procédé selon la revendication 5, dans lequel un seul acide aminé est délété de la séquence d'acides aminés du polypeptide cible.

7. Procédé selon l'une quelconque des revendications 3 à 6, comprenant les étapes suivantes :
a) la réalisation d'une réaction de transposition comprenant le mélange du transposon, de l'ADN cible et d'une enzyme transposase ;
b) la digestion de l'ADN obtenu au point (a) en utilisant une première enzyme de restriction qui reconnaît la première séquence de reconnaissance d'enzyme de restriction contenue dans le transposon ;
c) la séparation de l'ADN qui ne comprend pas le transposon ;
d) la réalisation d'une réaction de ligature intramoléculaire de l'ADN obtenu au point (c) ; et
e) l'expression d'une protéine à partir de l'ADN obtenu au point (d).

8. Procédé selon la revendication 1 ou 2, dans lequel au moins un acide aminé de la séquence d'acides aminés du polypeptide cible est substitué par un acide aminé différent.

9. Procédé selon la revendication 8, dans lequel un seul acide aminé de la séquence d'acides aminés du polypeptide cible est substitué par un acide aminé différent.

10. Procédé selon la revendication 8 ou 9, comprenant les étapes suivantes :
a) la réalisation d'une réaction de transposition comprenant le mélange du transposon, de l'ADN cible et d'une enzyme transposase ;
b) la digestion de l'ADN obtenu au point (a) en utilisant une première enzyme de restriction qui reconnaît la première séquence de reconnaissance d'enzyme de restriction contenue dans le transposon ;
c) la séparation de l'ADN qui ne comprend pas le transposon ;
d) la réalisation d'une ligature intermoléculaire de l'ADN obtenu au point (c) avec une deuxième séquence d'ADN comprenant au moins deux sites de reconnaissance pour une seconde enzyme de restriction situés de manière qu'au moins l'un des sites de clivage ne soit pas à une extrémité de la deuxième séquence d'ADN ;
e) la réalisation de la transformation d'un organisme hôte avec l'ADN obtenu au point (d) et la sélection des cellules contenant la deuxième séquence d'ADN ;
f) l'isolement de l'ADN à partir des cellules sélectionnées au point (e) et la digestion de cet ADN en utilisant une seconde enzyme de restriction qui reconnaît les sites de reconnaissance pour les secondes enzymes de restriction, la seconde enzyme de restriction réalisant une coupure externe ;
g) la réalisation d'une ligature intramoléculaire de l'ADN obtenu au point (f); et
h) l'expression d'une protéine à partir de l'ADN obtenu au point (g).

11. Procédé selon la revendication 10, dans lequel la seconde enzyme de restriction est identique à la première enzyme de restriction.

12. Procédé selon la revendication 10 ou 11, dans lequel la deuxième séquence d'ADN comprend un gène qui confère à une cellule hôte contenant la deuxième séquence d'ADN une caractéristique de sélection en comparaison d'une cellule qui ne contient pas la deuxième séquence d'ADN.

13. Procédé selon la revendication 1 ou 2, dans lequel la séquence d'acides aminés du polypeptide cible est altérée par l'insertion d'une autre séquence d'acides aminés.

14. Procédé selon la revendication 13 comprenant les étapes suivantes :
a) la réalisation d'une réaction de transposition comprenant le mélange du transposon, de l'ADN cible et d'une enzyme transposase ;
b) la digestion de l'ADN obtenu au point (a) en utilisant une première enzyme de restriction qui reconnaît la première séquence de reconnaissance d'enzyme de restriction contenue dans le transposon ;
c) la séparation de l'ADN qui ne comprend pas le transposon ;
d) la réalisation d'une ligature intermoléculaire de l'ADN obtenu au point (c) avec une troisième séquence d'ADN codant pour une autre séquence d'acides aminés ; et
e) l'expression d'une protéine à partir de l'ADN obtenu au point (d).

15. Procédé selon la revendication 13 ou 14, dans lequel la séquence d'acides aminés supplémentaire est une protéine entière, un domaine de protéine ou un fragment de protéine.

16. Procédé selon la revendication 15, dans lequel le fragment de protéine est un épitope.

17. Procédé selon la revendication 15, dans lequel le fragment de protéine est un domaine de liaison.

18. Procédé selon la revendication 15, dans lequel le fragment de protéine est un site allostérique.

19. Procédé selon la revendication 15, dans lequel le fragment de protéine est une région fonctionnelle définie.

20. Procédé selon la revendication 15, dans lequel le fragment de protéine est une interface d'oligomérisation.

21. Procédé selon l'une quelconque des revendications 14 à 20, dans lequel la troisième séquence ADN comprend un gène qui confère à une cellule hôte contenant la troisième séquence d'ADN une caractéristique de sélection en comparaison d'une cellule qui ne contient pas la troisième séquence d'ADN.

22. Procédé selon l'une quelconque des revendications 14 à 21, dans lequel la troisième séquence d'ADN présente un cadre ouvert de lecture qui est le même que celui de l'ADN cible.

23. Procédé selon l'une quelconque des revendications 14 à 22, dans lequel la troisième séquence d'ADN contient un codon d'arrêt.

24. Procédé selon l'une quelconque des revendications 14 à 23, dans lequel la troisième séquence d'ADN contient un codon d'initiation.

25. Procédé selon l'une quelconque des revendications précédentes, dans lequel la première enzyme de restriction est un enzyme de type IIS.

26. Procédé selon la revendication 25, dans lequel la première enzyme de restriction est l'enzyme *Mly*I.

27. Procédé selon l'une quelconque des revendications précédentes, dans lequel le transposon présente une faible préférence de site cible.

28. Procédé selon la revendication 27, dans lequel le transposon est dérivé de l'un des éléments suivants : mini-Mu, AT-2 ou Tn5.

29. Procédé selon l'une quelconque des revendications précédentes, dans lequel le transposon comprend un gène qui confère à une cellule hôte contenant le transposon une caractéristique de sélection en comparaison d'une cellule qui ne contient pas le transposon.

30. Procédé selon la revendication 27, dans lequel le transposon comprend la séquence d'ADN 5'-NGACTC-3' comme extrémité 5'-terminale et la séquence 5'-GAGTCN-3' comme extrémité 3'-terminale, ou comprend la séquence d'ADN 5'-NNNNGACTC-3' comme extrémité 5'-terminale et la séquence 5'-GAGTCNNNN-3' comme extrémité 3'-terminale, ou comprend la séquence d'ADN 5'-TGTTGACTC-3' comme extrémité 5'-terminale et la séquence 5'-GAGTCAACA-3' comme extrémité 3'-terminale, ou comprend la séquence d'ADN 5'-CTGACTC-3' comme extrémité 5'-terminale et la séquence 5'-GAGTCAG-3' comme extrémité 3'-terminale.

31. Procédé selon l'une quelconque des revendications précédentes, dans lequel l'ADN cible est véhiculé dans un plasmide.

32. Procédé selon la revendication 31, dans lequel le plasmide est le plasmide pNOM ou l'un de ses dérivés.

33. Transposon comprenant une séquence de reconnaissance pour une enzyme de restriction au niveau de chacune de ses extrémités terminales, la séquence de reconnaissance n'étant pas présente dans la partie restante du transposon, étant une séquence de reconnaissance pour une enzyme de restriction qui réalise une coupure externe, et étant placée de manière que l'enzyme de restriction ait un site de clivage d'ADN situé au-delà de l'extrémité terminale du transposon.

34. Transposon selon la revendication 33, approprié pour un usage dans un procédé selon l'une quelconque des revendications 1 à 32.

35. Transposon selon la revendication 33 ou 34, dans lequel chaque séquence de reconnaissance d'enzyme de restriction est située à une distance de 1 à 20 nucléotides par rapport à une extrémité terminale du transposon.

36. Transposon selon la revendication 35, dans lequel chaque séquence de reconnaissance d'enzyme de restriction est située à une distance de 1, 2, 3, 4 ou 5 nucléotides par rapport à une extrémité terminale du transposon.

37. Transposon selon l'une quelconque des revendications 33 à 36, dans lequel l'enzyme de restriction est l'enzyme *Mly*I.

38. Transposon selon la revendication 37, comprenant la séquence d'ADN 5'-NGACTC-3' comme extrémité 5'-terminale et la séquence 5'-GAGTCN-3' comme extrémité 3'-terminale.

39. Transposon selon la revendication 37, comprenant la séquence d'ADN 5'-NNNNGACTC-3' comme extrémité 5'-terminale et la séquence 5'-GAGTCNNNN-3' comme extrémité 3'-terminale.

40. Transposon selon la revendication 37, comprenant la séquence d'ADN 5'-TGTTGACTC-3' comme extrémité 5'-terminale et la séquence 5'-GAGTCAACA-3' comme extrémité 3'-terminale.

41. Transposon selon la revendication 37, comprenant la séquence d'ADN 5'-CTGACTC-3' comme extrémité 5'-terminale et la séquence 5'-GAGTCAG-3' comme extrémité 3'-terminale.

42. Transposon selon la revendication 40 ou 41, comprenant au moins une variation dans la séquence ADN 5'-terminale et/ou 3'-terminale, le transposon étant viable pour une réaction de transposition.

43. Plasmide présentant la séquence d'ADN représentée sur la Fig. 1.

44. Plasmide présentant une séquence d'ADN qui a été adaptée à partir de la séquence représentée sur la Fig. 1 par le biais :
a) de mutations silencieuses effectuées dans la séquence d'ADN,
b) d'une substitution du gène *bla* par un autre marqueur de sélection,
c) de l'altération d'élément non essentiels de la séquence d'ADN, ou
d) de l'insertion d'une séquence d'ADN supplémentaire intéressante à un endroit de la séquence d'ADN représentée sur la Fig. 1.

45. Trousse comprenant un transposon selon l'une quelconque des revendications 33 à 42.

46. Trousse selon la revendication 45, comprenant en outre un plasmide selon la revendication 43 ou 44.

47. Trousse selon la revendication 45 ou 46 comprenant également une transposase.

48. Trousse selon l'une quelconque des revendications 45 à 47, comprenant encore au moins un tampon.

49. Trousse selon l'une quelconque des revendications 45 à 48, comprenant, en outre, au moins un oligonucléotide.

50. Trousse selon l'une quelconque des revendications 45 à 49, pour une utilisation dans le procédé selon l'une quelconque des revendications 1 à 32.

51. Procédé consistant à déterminer si l'introduction d'une mutation dans un polypeptide cible altère une activité détectable de ce polypeptide, comprenant le procédé selon l'une quelconque des revendications 1 à 32, ainsi que les étapes supplémentaires suivantes consistant à :
a) cribler pour une différence dans l'activité du polypeptide cible altéré en comparaison du polypeptide cible non altéré ; et
b) séquencer le polypeptide cible altéré pour déterminer la position de l'insertion, de la délétion ou de la substitution d'acides aminés.


### SEQUENCE LISTING

<110> University College Cardiff Consultants Ltd
<120> Polypeptide mutagenesis method
<130> P105787PCT
<150> GB0501189.5
   <151> 2005-01-20
<160> 100
<170> PatentIn version 3.2
<210> 1
   <211> 6
   <212> DNA
   <213> Artificial
<220>
   <223> Transposon terminal
<220>
   <221> misc_feature
   <222> (1) .. (1)
   <223> n is a, c, g, or t
<400> 1
   ngactc 6
<210> 2
   <211> 6
   <212> DNA
   <213> Artificial
<220>
   <223> Transposon terminal
<220>
   <221> misc_feature
   <222> (6) .. (6)
   <223> n is a, c, g, or t
<400> 2
   gagtcn 6
<210> 3
   <211> 12
   <212> DNA
   <213> Artificial
<220>
   <223> Transposon terminal
<400> 3
   tgactcggcg ca 12
<210> 4
   <211> 12
   <212> DNA
   <213> Artificial
<220>
   <223> Transposon terminal
<400> 4
   tgcgccgagt ca 12
<210> 5
   <211> 9
   <212> DNA
   <213> Artificial
<220>
   <223> Transposon terminal
<220>
   <221> misc_feature
   <222> (1)..(4)
   <223> n is a, c, g, or t
<400> 5
   nnnngactc 9
<210> 6
   <211> 9
   <212> DNA
   <213> Artificial
<220>
   <223> Transposon terminal
<220>
   <221> misc_feature
   <222> (6) .. (9)
   <223> n is a, c, g, or t
<400> 6
   gagtcnnnn 9
<210> 7
   <211> 12
   <212> DNA
   <213> Artificial
<220>
   <223> Transposon terminal
<400> 7
   tgaagactcg ca 12
<210> 8
   <211> 12
   <212> DNA
   <213> Artificial
<220>
   <223> Transposon terminal
<400> 8
   tgcgagtctt ca 12
<210> 9
   <211> 9
   <212> DNA
   <213> Artificial
<220>
   <223> Transposon terminal
<400> 9
   tgttgactc 9
<210> 10
   <211> 9
   <212> DNA
   <213> Artificial
<220>
   <223> Transposon terminal
<400> 10
   gagtcaaca 9
<210> 11
   <211> 19
   <212> DNA
   <213> Artificial
<220>
   <223> Transposon terminal
<400> 11
   ctgactc 7
<210> 12
   <211> 19
   <212> DNA
   <213> Artificial
<220>
   <223> Transposon terminal
<400> 12
   gagtcag 7
<210> 13
   <211> 33
   <212> DNA
   <213> Artificial
<220>
   <223> Primer sequence
<400> 13
   gaaactcgag agacgaaagg gcctcgtgat acg 33
<210> 14
   <211> 37
   <212> DNA
   <213> Artificial
<220>
   <223> Primer sequence
<400> 14
   catccatagt tgcctgactg cccgtcgtgt agataac 37
<210> 15
   <211> 37
   <212> DNA
   <213> Artificial
<220>
   <223> Primer sequence
<400> 15
   gttatctaca cgacgggcag tcaggcaact atggatg 37
<210> 16
   <211> 17
   <212> DNA
   <213> Artificial
<220>
   <223> Primer sequence
<400> 16
   ccaacccggt aagacac 17
<210> 17
   <211> 39
   <212> DNA
   <213> Artificial
<220>
   <223> Primer sequence
<220>
   <221> misc_feature
   <222> (18)..(19)
   <223> n is a, c, g, or t
<400> 17
   gtgtcttacc gggttggnnt caagacgata gttaccgga 39
<210> 18
   <211> 42
   <212> DNA
   <213> Artificial
<220>
   <223> Primer sequence
<400> 18
   cttcctcgct catatgctcg ctgcgctcgg tcgttcggct gc 42
<210> 19
   <211> 19
   <212> DNA
   <213> Artificial
<220>
   <223> Primer sequence
<400> 19
   atgcgtccgg cgtagagga 19
<210> 20
   <211> 21
   <212> DNA
   <213> Artificial
<220>
   <223> Primer sequence
<400> 20
   gctagttatt gctcagcggt g 21
<210> 21
   <211> 36
   <212> DNA
   <213> Artificial
<220>
   <223> Primer sequence
<400> 21
   gcttagatct gactcggcgc acgaaaaacg cgaaag 36
<210> 22
   <211> 21
   <212> DNA
   <213> Artificial
<220>
   <223> Primer sequence
<400> 22
   agctggcgaa agggggatgt g 21
<210> 23
   <211> 26
   <212> DNA
   <213> Artificial
<220>
   <223> Primer sequence
<400> 23
   ttatgcttcc ggctcgtatg ttgtgt 26
<210> 24
   <211> 24
   <212> DNA
   <213> Artificial
<220>
   <223> Primer sequence
<400> 24
   tccgctcatg agacaataac cctg 24
<210> 25
   <211> 22
   <212> DNA
   <213> Artificial
<220>
   <223> Primer sequence
<400> 25
   ctacggggtc tgacgctcag tg 22
<210> 26
   <211> 12
   <212> DNA
   <213> Artificial
<220>
   <223> Transposon insertion
<400> 26
   gggttttttc cc 12
<210> 27
   <211> 12
   <212> DNA
   <213> Artificial
<220>
   <223> Transposon insertion
<400> 27
   gggtttcttc cc 12
<210> 28
   <211> 12
   <212> DNA
   <213> Artificial
<220>
   <223> Transposon insertion
<400> 28
   gggtttcctc cc 12
<210> 29
   <211> 4
   <212> PRT
   <213> Artificial
<220>
   <223> Protein insertion
<400> 29
   Gly Phe Phe Pro
   1
<210> 30
   <211> 4
   <212> PRT
   <213> Artificial
<220>
   <223> Protein insertion
<400> 30
   Gly Phe Leu Pro
   1
<210> 31
   <211> 4
   <212> PRT
   <213> Artificial
<220>
   <223> Protein insertion
<400> 31
   Gly Phe Pro Pro
   1
<210> 32
   <211> 12
   <212> DNA
   <213> Escherichia coli
<400> 32
   cgccccgaag aa 12
<210> 33
   <211> 12
   <212> DNA
   <213> Escherichia coli
<400> 33
   ttatcccgta tt 12
<210> 34
   <211> 12
   <212> DNA
   <213> Escherichia coli
<400> 34
   catcttacgg at 12
<210> 35
   <211> 12
   <212> DNA
   <213> Escherichia coli
<400> 35
   catcttacgg at 12
<210> 36
   <211> 12
   <212> DNA
   <213> Escherichia coli
<400> 36
   gacgagcgtg ac 12
<210> 37
   <211> 12
   <212> DNA
   <213> Escherichia coli
<400> 37
   ttaactggcg aa 12
<210> 38
   <211> 12
   <212> DNA
   <213> Escherichia coli
<400> 38
   gttgcaggac ca 12
<210> 39
   <211> 12
   <212> DNA
   <213> Escherichia coli
<400> 39
   gatgaacgaa at 12
<210> 40
   <211> 4
   <212> PRT
   <213> Escherichia coli
<400> 40
   Arg Pro Glu Glu
   1
<210> 41
   <211> 4
   <212> PRT
   <213> Escherichia coli
<400> 41
   Leu Ser Arg Ile
   1
<210> 42
   <211> 4
   <212> PRT
   <213> Escherichia coli
<400> 42
   His Leu Thr Asp
   1
<210> 43
   <211> 4
   <212> PRT
   <213> Escherichia coli
<400> 43
   Asp Glu Arg Asp
   1
<210> 44
   <211> 4
   <212> PRT
   <213> Escherichia coli
<400> 44
   Leu Thr Gly Glu
   1
<210> 45
   <211> 4
   <212> PRT
   <213> Escherichia coli
<400> 45
   Val Ala Gly Pro
   1
<210> 46
   <211> 4
   <212> PRT
   <213> Escherichia coli
<400> 46
   Asp Glu Arg Asn
   1
<210> 47
   <211> 30
   <212> DNA
   <213> Artificial
<220>
   <223> Primer sequence
<400> 47
   cgaccgactc aatacctgtg acggaagatc 30
<210> 48
   <211> 32
   <212> DNA
   <213> Artificial
<220>
   <223> Primer sequence
<220>
   <221> misc_feature
   <222> (1) .. (3)
   <223> n is a, c, g, or t
<400> 48
   nnnaactgga ctcaggcatt tgagaagcac ac 32
<210> 49
   <211> 12
   <212> DNA
   <213> Escherichia coli
<400> 49
   cacaacatgg gg 12
<210> 50
   <211> 12
   <212> DNA
   <213> Escherichia coli
<400> 50
   cagatcgctg ag 12
<210> 51
   <211> 12
   <212> DNA
   <213> Escherichia coli
<400> 51
   acgatgcctg ta 12
<210> 52
   <211> 12
   <212> DNA
   <213> Escherichia coli
<400> 52
   gcttcccggc aa 12
<210> 53
   <211> 12
   <212> DNA
   <213> Escherichia coli
<400> 53
   atgcctgtag ca 12
<210> 54
   <211> 12
   <212> DNA
   <213> Escherichia coli
<400> 54
   gccataacca tg 12
<210> 55
   <211> 12
   <212> DNA
   <213> Escherichia coli
<400> 55 12
   gactggatgg ag 12
<210> 56
   <211> 12
   <212> DNA
   <213> Escherichia coli
<400> 56 12
   gctgaagatc ag 12
<210> 57
   <211> 12
   <212> DNA
   <213> Escherichia coli
<400> 57 12
   gagcaactcg gt 12
<210> 58
   <211> 12
   <212> DNA
   <213> Escherichia coli
<400> 58 12
   cagatcgctg ag 12
<210> 59
   <211> 12
   <212> DNA
   <213> Artificial
<220>
   <223> Substitution sequence
<400> 59 12
   cacaacacgc gg 12
<210> 60
   <211> 12
   <212> DNA
   <213> Artificial
<220>
   <223> Substitution sequence
<400> 60 12
   cagatcgcgt cg 12
<210> 61
   <211> 12
   <212> DNA
   <213> Artificial
<220>
   <223> Substitution sequence
<400> 61
   acgatgccat ca 12
<210> 62
   <211> 12
   <212> DNA
   <213> Artificial
<220>
   <223> Substitution sequence
<400> 62
   gcttccgctc aa 12
<210> 63
   <211> 12
   <212> DNA
   <213> Artificial
<220>
   <223> Substitution sequence
<400> 63
   atgcccagag ca 12
<210> 64
   <211> 12
   <212> DNA
   <213> Artificial
<220>
   <223> Substitution sequence
<400> 64
   gccataaact tg 12
<210> 65
   <211> 12
   <212> DNA
   <213> Artificial
<220>
   <223> Substitution sequence
<400> 65
   gactggacta ag 12
<210> 66
   <211> 12
   <212> DNA
   <213> Artificial
<220>
   <223> Substitution sequence
<400> 66
   gctgaagagt ag 12
<210> 67
   <211> 12
   <212> DNA
   <213> Artificial
<220>
   <223> Substitution sequence
<400> 67
   gagcaacaac gt 12
<210> 68
   <211> 12
   <212> DNA
   <213> Artificial
<220>
   <223> Substitution sequence
<400> 68
   cagatcgcgt cg 12
<210> 69
   <211> 23
   <212> DNA
   <213> Artificial
<220>
   <223> Primer sequence
<400> 69
   gcagatcttg aagacaatat gga 23
<210> 70
   <211> 32
   <212> DNA
   <213> Artificial
<220>
   <223> Primer sequence
<400> 70
   ggcggtagcg cagatcttga agacaatatg ga 32
<210> 71
   <211> 23
   <212> DNA
   <213> Artificial
<220>
   <223> Primer sequence
<400> 71 23
   cctatacttc tggtgatagg cgt 23
<210> 72
   <211> 32
   <212> DNA
   <213> Artificial
<220>
   <223> Primer sequence
<400> 72
   gctgccaccc ctatacttct ggtgataggc gt 32
<210> 73
   <211> 24
   <212> DNA
   <213> Artificial
<220>
   <223> Primer sequence
<400> 73 24
   cgcagatctt gaagacaata tgga 24
<210> 74
   <211> 33
   <212> DNA
   <213> Artificial
<220>
   <223> Primer sequence
<400> 74
   cggcggtagc gcagatcttg aagacaatat gga 33
<210> 75
   <211> 22
   <212> DNA
   <213> Artificial
<220>
   <223> Primer sequence
<400> 75
   ctatacttct ggtgataggc gt 22
<210> 76
   <211> 31
   <212> DNA
   <213> Artificial
<220>
   <223> Primer sequence
<400> 76
   ctgccacccc tatacttctg gtgataggcg t 31
<210> 77
   <211> 25
   <212> DNA
   <213> Artificial
<220>
   <223> Primer sequence
<400> 77
   ctgcagatct tgaagacaat atgga 25
<210> 78
   <211> 34
   <212> DNA
   <213> Artificial
<220>
   <223> Primer sequence
<400> 78
   ctggcggtag cgcagatctt gaagacaata tgga 34
<210> 79
   <211> 21
   <212> DNA
   <213> Artificial
<220>
   <223> Primer sequence
<400> 79
   tatacttctg gtgataggcg t 21
<210> 80
   <211> 30
   <212> DNA
   <213> Artificial
<220>
   <223> Primer sequence
<400> 80
   tgccacccct atacttctgg tgataggcgt 30
<210> 81
   <211> 11
   <212> DNA
   <213> Escherichia coli
<400> 81
   gaaaagcatc t 11
<210> 82
   <211> 11
   <212> DNA
   <213> Escherichia coli
<400> 82
   aaactattaa c 11
<210> 83
   <211> 11
   <212> DNA
   <213> Escherichia coli
<400> 83
   catcttacgg a 11
<210> 84
   <211> 11
   <212> DNA
   <213> Escherichia coli
<400> 84
   acgacgggca g 11
<210> 85
   <211> 11
   <212> DNA
   <213> Escherichia coli
<400> 85
   ccggagctga a 11
<210> 86
   <211> 11
   <212> DNA
   <213> Escherichia coli
<400> 86
   catcttacgg a 11
<210> 87
   <211> 10
   <212> DNA
   <213> Escherichia coli
<400> 87
   aagcatctta 10
<210> 88
   <211> 10
   <212> DNA
   <213> Escherichia coli
<400> 88
   aagcatctta 10
<210> 89
   <211> 39
   <212> DNA
   <213> Artificial
<220>
   <223> Primer sequence
<400> 89
   ctgactctta tacacaagtc gcgaaagcgt ttcacgata 39
<210> 90
   <211> 39
   <212> DNA
   <213> Artificial
<220>
   <223> Primer sequence
<400> 90
   ctgagtctta tacacatctc gcgaaagcgt ttcacgata 39
<210> 91
   <211> 4
   <212> PRT
   <213> Artificial
<220>
   <223> Protein insertion
<400> 91
   Gly Tyr Tyr Ile
   1
<210> 92
   <211> 4
   <212> PRT
   <213> Artificial
<220>
   <223> Protein insertion
<400> 92
   Cys Gly Gly Ala
   1
<210> 93
   <211> 4
   <212> PRT
   <213> Artificial
<220>
   <223> Protein insertion
<400> 93
   Gly Ala Ala Val
   1
<210> 94
   <211> 4
   <212> PRT
   <213> Artificial
<220>
   <223> Protein insertion
<400> 94
   Val Leu Leu Ser
   1
<210> 95
   <211> 4
   <212> PRT
   <213> Artificial
<220>
   <223> Protein insertion
<400> 95
   Glu Leu Leu Cys
   1
<210> 96
   <211> 4
   <212> PRT
   <213> Artificial
<220>
   <223> Protein insertion
<400> 96
   Ser Pro Arg Gly
   1
<210> 97
   <211> 4
   <212> PRT
   <213> Artificial
<220>
   <223> Protein insertion
<400> 97
   Ala Leu Leu Gly
   1
<210> 98
   <211> 4
   <212> PRT
   <213> Artificial
<220>
   <223> Protein insertion
<400> 98
   Leu Gly Gly Pro
   1
<210> 99
   <211> 4
   <212> PRT
   <213> Artificial
<220>
   <223> Protein insertion
<400> 99
   Pro Ser Ser Arg
   1
<210> 100
   <211> 2115
   <212> DNA
   <213> Artificial
<220>
   <223> Plasmid sequence
<400> 100
   ctgctgccag tggcgataag tcgtgtctta ccgggttggg ttcaagacga tagttaccgg   60
   ataaggcgca gcggtcgggc tgaacggggg gttcgtgcac acagcccagc ttggagcgaa  120
   cgacctacac cgaactgaga tacctacagc gtgagctatg agaaagcgcc acgcttcccg  180
   aagggagaaa ggcggacagg tatccggtaa gcggcagggt cggaacagga gagcgcacga  240
   gggagcttcc agggggaaac gcctggtatc tttatagtcc tgtcgggttt cgccacctct  300
   gacttgagcg tcgatttttg tgatgctcgt caggggggcg gagcctatgg aaaaacgcca  360
   gcaacgcggc ctttttacgg ttcctggcct tttgctggcc ttttgctcac atgttctttc  420
   ctgcgttatc ccctgattct gtggataacc gtattaccgc ctttgagtga gctgataccg 480
   ctcgccgcag ccgaacgacc gagcgcagcg agcatatgaa atacctgctg ccgaccgctg 540
   ctgctggtct gctgctcctc gctgcccagc cggcgatggc catggatatc ggaattaatt 600
   cggatccgaa ttcgagctcc gtcgacaagc ttgcggccgc actcgagaga cgaaagggcc 660
   tcgtgatacg cctattttta taggttaatg tcatgataat aatggtttct tagacgtcag 720
   gtggcacttt tcggggaaat gtgcgcggaa cccctatttg tttatttttc taaatacatt 780
   caaatatgta tccgctcatg agacaataac cctgataaat gcttcaataa tattgaaaaa 840
   ggaagagtat gagtattcaa catttccgtg tcgcccttat tccctttttt gcggcatttt 900
   gccttcctgt ttttgctcac ccagaaacgc tggtgaaagt aaaagatgct gaagatcagt 960
   tgggtgcacg agtgggttac atcgaactgg atctcaacag cggtaagatc cttgagagtt 1020
   ttcgccccga agaacgtttt ccaatgatga gcacttttaa agttctgcta tgtggcgcgg 1080
   tattatcccg tattgacgcc gggcaagagc aactcggtcg ccgcatacac tattctcaga 1140
   atgacttggt tgagtactca ccagtcacag aaaagcatct tacggatggc atgacagtaa 1200
   gagaattatg cagtgctgcc ataaccatga gtgataacac tgcggccaac ttacttctga 1260
   caacgatcgg aggaccgaag gagctaaccg cttttttgca caacatgggg gatcatgtaa 1320
   ctcgccttga tcgttgggaa ccggagctga atgaagccat accaaacgac gagcgtgaca 1380
   ccacgatgcc tgtagcaatg gcaacaacgt tgcgcaaact attaactggc gaactactta 1440
   ctctagcttc ccggcaacaa ttaatagact ggatggaggc ggataaagtt gcaggaccac 1500
   ttctgcgctc ggcccttccg gctggctggt ttattgctga taaatctgga gccggtgagc 1560
   gtgggtctcg cggtatcatt gcagcactgg ggccagatgg taagccctcc cgtatcgtag 1620
   ttatctacac gacggggagt caggcaacta tggatgaacg aaatagacag atcgctgaga 1680
   taggtgcctc actgattaag cattggtaac tgtcagacca agtttactca tatatacttt 1740
   agattgattt aaaacttcat ttttaattta aaaggatcta ggtgaagatc ctttttgata 1800
   atctcatgac caaaatccct taacgtgagt tttcgttcca ctgagcgtca gaccccgtag 1860
   aaaagatcaa aggatcttct tgagatcctt tttttctgcg cgtaatctgc tgcttgcaaa 1920
   caaaaaaacc accgctacca gcggtggttt gtttgccgga tcaagagcta ccaactcttt 1980
   ttccgaaggt aactggcttc agcagagcgc agataccaaa tactgttctt ctagtgtagc 2040
   cgtagttagg ccaccacttc aagaactctg tagcaccgcc tacatacctc gctctgctaa 2100
   tcctgttacc agtgg 2115